# EUROPEAN PATENT APPLICATION

(11) **EP 3 689 420 A1**
(43) Date of publication of application: **05.08.2020**
(21) Application number: 19205481.5
(22) Date of filing: 23.01.2015
(51) Int. Cl.: A61P 35/00, C07D 311/30, C07D 311/62

(54) **ICARIIN DERIVATIVES FOR USE IN TREATING MYELODYSPLASIA SYNDROME**

(30) Priority: 23.01.2014 US 201461930757 P; 10.04.2014 US 201461977985 P
(62) Divisional of application: 15740729.7
(71) Applicant: H. Lee Moffitt Cancer Center & Research Institute, Inc., Tampa, Florida 33612 (US)
(72) Inventor: WEI, Sheng, Tampa, FL 33647 (US); LIST, Alan, Tampa, FL 33647 (US); LAWRENCE, Nicholas, Tampa, FL 33647 (US)
(74) Representative: Dehns

(57) **Abstract**

Disclosed are compounds of Formula II, III-A, V-A or VI-A, as defined herein, for use in treating myelodysplastic syndrome.

## Description

### CROSS REFERENCE TO RELATED APPLICATIONS

This application claims the benefit of priority to U.S. Provisional Application 61/930,757, filed January 23, 2014, and U.S. Provisional Application 61/977,985, filed April 10, 2014, the disclosures of each are incorporated herein by referenced in their entirities.

### BACKGROUND

Inflammation is a hallmark of cancer and promotes the development and progression of cancer as well as the invasion of the immune system by tumor cells. Inflammation-induced cancer can be attributed to myeloid-derived suppressor cells (MDSCs), which accumulate in tumor bearing hosts, particularly in the local tumor microenvironment. MDSCs, characterized as Gr1⁺CD11b⁺ in mice and HLA-DR⁻Lin⁻CD33⁺ in humans, were identified as the major immune creator of an immunosuppressive and tumorigenic microenvironment (Gabrilovich DI, Nagaraj S. Nat Rev Immunol. 2009;9(3):162-74). In healthy individuals, these cells exist as immature myeloid cells (IMC) and are part of normal myelopoiesis as they can quickly differentiate into mature monocytes, DC and neutrophils. However, under certain pathological situations, including inflammation and cancer, these IMCs are activated and accumulate in local tissues where they act both as tumor promoting and immunosuppressive cells through the release of soluble angiogenic and suppressive factors, such as VEGF, TGFβ, IL-6, or IL-10. They can also directly suppress tumor-specific CD4⁺ and CD8⁺ T-cell responses and induce CD4⁺CD25⁺FOXP3⁺ regulatory T cells (Tregs). Moreover, they can also contribute directly to the pathogenesis of cancer and leukemia by preventing the maturation of bone marrow progenitor cells as well as modulating hematopoietic stem cell/progenitor cell development. Further, reactive oxygen and nitrogen species (ROS and RNS respectively) and active STAT3 are implicated in MDSC function and are closely associated with up-regulation of immunosuppressive cytokines and tumor promoting factors. Hence targeting MDSCs and their downstream effector mechanisms is essential to restore immune recognition of the tumor and inhibit cancer progression. However, there are currently no effective therapeutic strategies to contain them.

Human MDSCs are unique in lacking all lineage markers and are defined by only one key receptor, CD33; a well-known surface marker of immature myeloid cells. It represents a 67 kDa type 1 transmembrane sialo-glycoprotein also known as the prototypical member of a subset of Sialic acid-binding Ig super-family lectins (SIGLEC). This particular subgroup is known as the CD33-related SIGLECs (CD33-r Siglecs) where CD33 is functionally known as SIGLEC 3. In humans, there are nine SIGLECs related to CD33, including SIGLEC 3, 5 and 14, which share 50-99% homology. Notwithstanding this homology, each SIGLEC has a unique specificity for sialylated ligands, making it more probable that each protein mediates a distinct function. All SIGLECs have an amino-terminal variable V-set immunoglobulin domain that binds sialic acid and, although the sugar moiety they bind is known, their complete ligand is not known. Another characteristic property of CD33-r SIGLECs, including SIGLEC 3, is the presence of two conserved immune-receptor tyrosine-based inhibitory motifs (ITIM) in their cytoplasmic region. Engagement of SIGLEC 3 with anti-SIGLEC 3 antibody, or through its ligand, leads to the phosphorylation of these tyrosine motifs which recruit and activate Src homology-2 (SH2) domain-containing tyrosine phosphatases (SHP-1 and SHP-2) (Paul SP et al. Blood. 2000;96(2):483-90). Classically, receptors with ITIM domains function to suppress activation or maturation signals that emanate from receptors associated with activating motifs (ITAMs) through the recruitment of tyrosine and inositol phosphatases.

Additional CD33-r SIGLECs were discovered that deliver an activating, rather than inhibitory, signal. These alternative receptors lack ITIMs and instead interact with DAP12 (a DNAX-activating protein of 12kDa). This interaction occurs through a positively charged anionic residue located in the transmembrane domain of the receptor, which non-covalently binds to a negatively charged aspartic acid residue on DAP12. This adaptor molecule is an ITAM-bearing protein shared by the majority of NK activating receptors. Signaling through it leads to the activation of Syk protein tyrosine kinase, phosphoinositide 3-kinase (PI3K), and ERK/MAPK. DAP12 partners with activating receptors, including SIGLEC-14 in humans and SIGLEC-H in mice, and plays a role in myeloid development through their involvement in the maturation and differentiation of hematopoietic stem cell into monocytes as well as promotion of DC maturation and survival. Therefore, DAP12 can down-regulate MDSC function and increase population numbers by counteracting SIGLEC3-ITIM signaling and driving MDSC differentiation into mature cells.

Recently, SIGLEC3's endogenous ligand was identified. Using a SIGLEC3-IgG Fc chimeric fusion protein, mass spectrometry identified a protein to be S100A9. This is significant because S100A8 and S100A9 (also called myeloid-related protein (MRP)-8 and 14 or Calgranulin A and B, respectively) can be a potent inflammatory mediator of MDSC activation in tumor-bearers. Furthermore, it has been found that SIGLEC 3-expressing MDSCs isolated from MDS patients (Myelodysplastic syndrome, a premalignant disorder that transforms to AML (acute myeloid leukemia)) have a high capacity for disruption of normal hematopoiesis (Wei S, et al. ASH Annual Meeting Abstracts. 2009;114(22):597).

S100A8 and S100A9 (encoded by genes *S100A8* and *S100A9,* respectively) are calcium-binding proteins expressed in myeloid cells during specific stages of differentiation and they are recognized as endogenous damage-associated molecular patterns (DAMPs). Working as a heterodimer (called Calprotectin), S100A8/A9 acts as an effective endogenous mediator to promote inflammation and MDSC activation. Furthermore, they are released at sites of ongoing inflammation leading to increased serum levels and correlating with the degree of inflammation. Using mice devoid of functional S100A8/A9, it has been established that both proteins can activate Toll like receptor-4 (TLR4) and hence are involved in TLR4-mediated signaling to promote inflammation. Up-regulation of S100A8/A9 in MDSCs can play a role in inhibition of DC and macrophage differentiation and can induce accumulation of MDSCs that can contribute to cancer development and tumor spread. Not only can S100A8 and S100A9 be related to the *in vivo* increase in the number of MDSCs in tumor-bearing mice but they can also be related to the inhibitory effects on myeloid cell differentiation. This idea was supported by S100A9 knock out mice that presented normal myeloid cell differentiation and greatly reduced MDSCs. In contrast, MDSC accumulation was enhanced in S100A9 transgenic mice (Tg) with inhibition of macrophage and DC differentiation (Cheng P et al. J Exp Med. 2008;205(10):2235-49).

Given the current lack of effective targeted therapies to MDSC in cancer, along with their role in other inflammation associated diseases, inhibitors of MDSC are desireable. The compounds, compositions and methods disclosed herein address these and other needs.

### SUMMARY

In accordance with the purposes of the disclosed compounds, compositions and methods, as embodied and broadly described herein, the disclosed subject matter relates to compounds, compositions and methods of making and using the compositions. In more specific aspects, the disclosed subject matter relates to compounds that are derivatives of Icariin, methods of using the compounds, and compositions comprising the compounds. In certain aspects, the disclosed subject matter relates to compounds having the chemical structure shown in Formulas I-VIII, as defined herein. In still further aspects, the disclosed subject matter relates to methods for treating precancerous syndromes in a subject. For example, disclosed herein are methods whereby an effective amount of a compound or composition disclosed herein is administered to a subject having a precancerous syndrome, for example myelodisplastic syndrome, and who is in need of treatment thereof.

Additional advantages will be set forth in part in part in the description that follows and the Figures, and in part will be obvious from the description, or may be learned by practice of the aspects described below. The advantages described below will be realized and attained by means of the elements and combinations particularly pointed out in the appended claims. It is to be understood that both the foregoing general description and the following detailed description are exemplary and explanatory only and are not restrictive.

### DESCRIPTION OF FIGURES

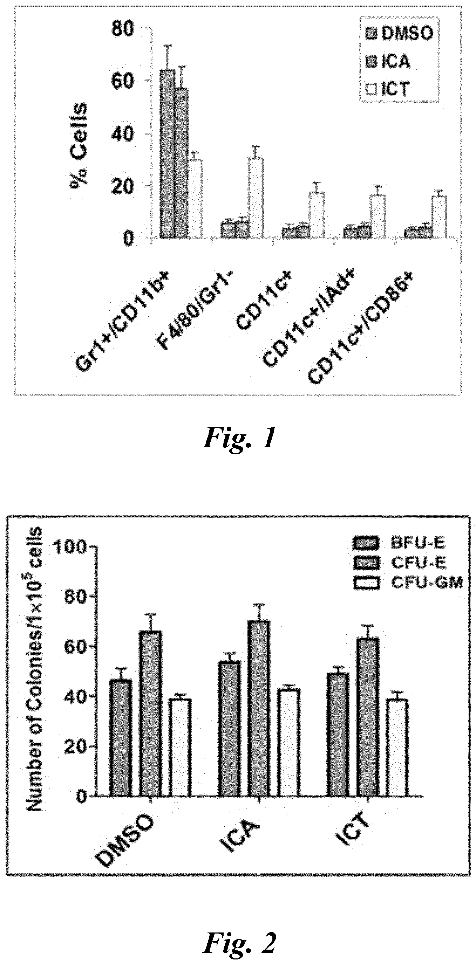

**Fig. 1** depicts the effects of ICA or ICT on the differentiation of MDSC. MDSC were isolated from spleens of 4T1 tumor-bearing mice. The maturation markers indicated were used to evaluate the differentiation and accumulation of MDSC in tumor bearing mice. Cell phenotype was evaluated by flow cytometry.
**Fig. 2** depicts the effect, or lack thereof, of ICA and ICT on hematopoiesis. BMNCs from three healthy donors were treated with ICA or ICT for 48 hours after which colony formation was assessed using MethoCult H4434 complete medium with cytokines. CFU-E, BFU-E and CFU-GM were indentified and counted using an inverted light microscope. Each bar represents the mean results of three individuals with duplicate plates. Results are expressed as means ± SD.

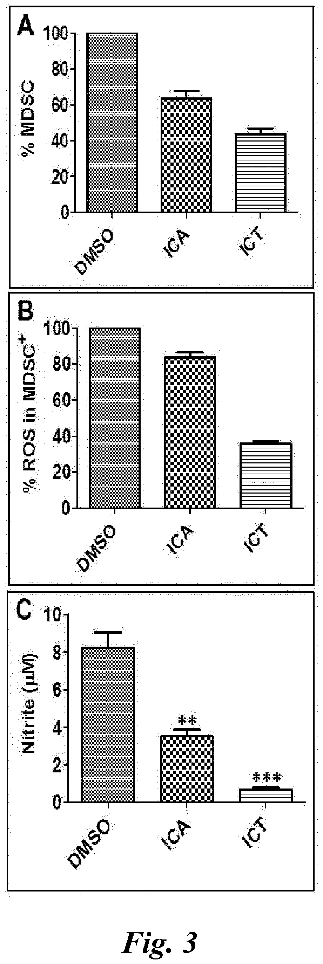

**Fig. 3** depicts the effect of ICA and ICT on expansion of MDSCs isolated from tumor and their ROS and NO production. Gr1⁺ cells were isolated from tumors of 4T1 tumor-bearing mice. A) MDSCs were treated with 20 µM of ICA, ICT, or DMSO for 48h. Bars represent the relative percentage of MDSC as compared to DMSO treated cells measured by flow cytometry. B) Production of ROS and C) NO activity of purified MDSC from tumor tissue.

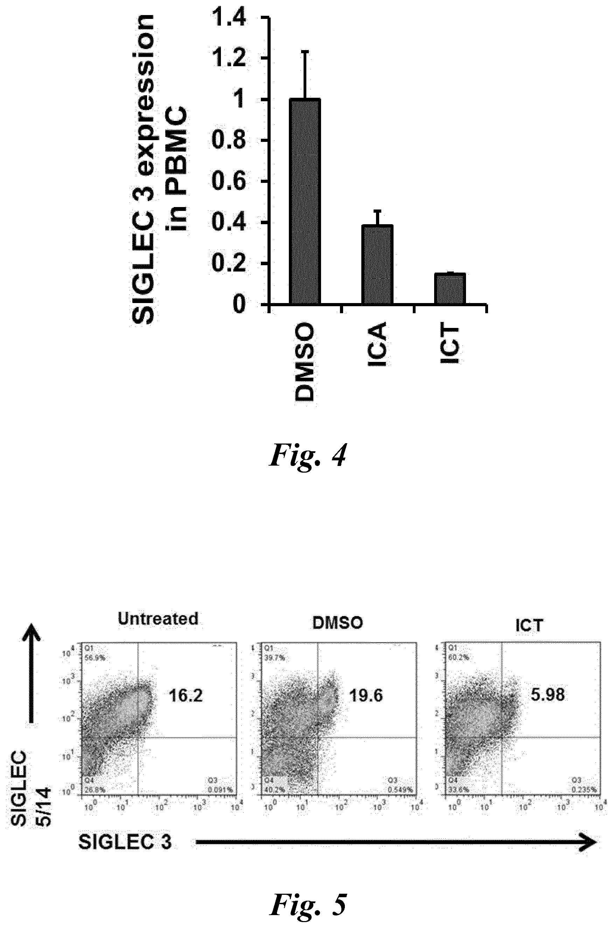

**Fig. 4** depicts the down-regulation of the RNA expression of SIGLEC3 on PBMCs due to ICA and ICT. Healthy PBMC were cultured for 48 hours with 1 µM of DMSO, ICA or ICT and the expression of SIGLEC3 was measured by Q-PCR and analyzed by the ΔΔCt method. Error bars indicate SEM.
**Fig. 5** depicts the effect of ICT on the expression of SIGLEC3 and SIGLEC5 on PBMC. PBMC from healthy donors were treated for 48 hours with DMSO or ICT and stained extra-cellularly with SIGLEC3-PE and SIGLEC5/14-APC antibodies (BD biosciences).

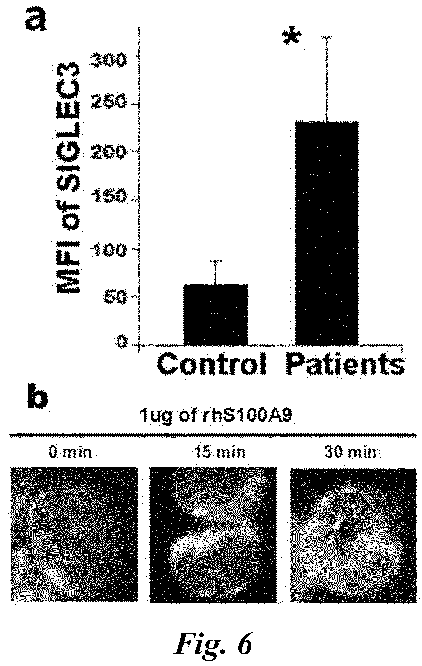

**Fig. 6** depicts the effect of MDSC from MDS BM on SIGLEC3 and S100A9 binding. a) MDSC from MDS patients (n=12) and their age-matched healthy controls (n=8) were analyzed by flow cytometry for the Mean fluorescence intensity (MFI) of SIGLEC3 surface expression, * *P<* 0.0005. b) MDSCs from the BM of MDS patients were treated with human recombinant S100A9-DDK. After washing cells were cytospined and stained for SIGLEC3 (FITC) and biotinylated anti-DDK (APC).

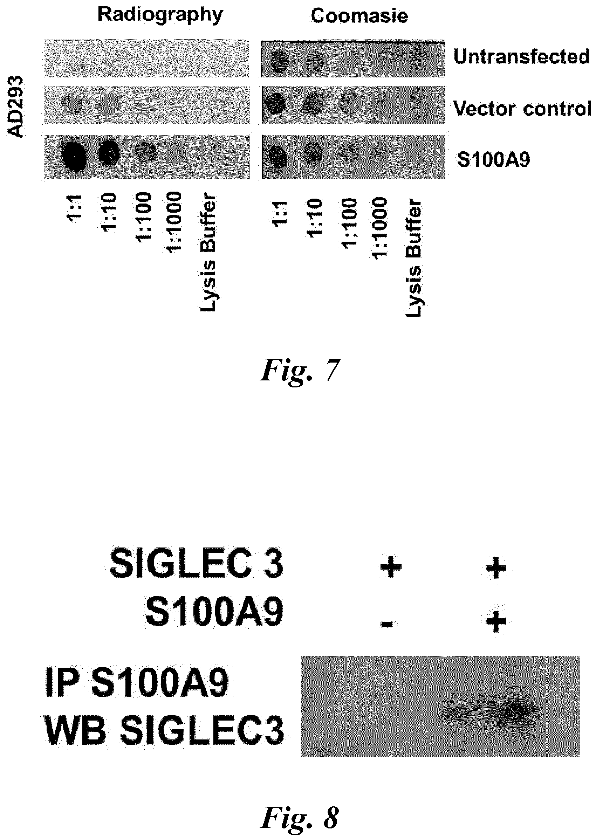

**Fig. 7** depicts a dot blot with chimeric SIGLEC receptor IgG-Fc molecules. S100A9 transfected AD293 lysate were serially diluted onto a nitrocellulose membrane starting at 30 µg and incubated with the SIGLEC3 chimera. Membranes were blotted with anti-human IgG-HRP. Coomasie staining served as loading control.
**Fig. 8** depicts the association of SIGLEC3 with S100A9. SJCRH30 cells were transfected with construct as indicated for 72h (allowing S100A9 released in an autocrine fashion binding to SIGLEC 3). Immunoprecipitation was performed with S100A9 antibody followed by Western Blot with SIGLEC 3 antibody.

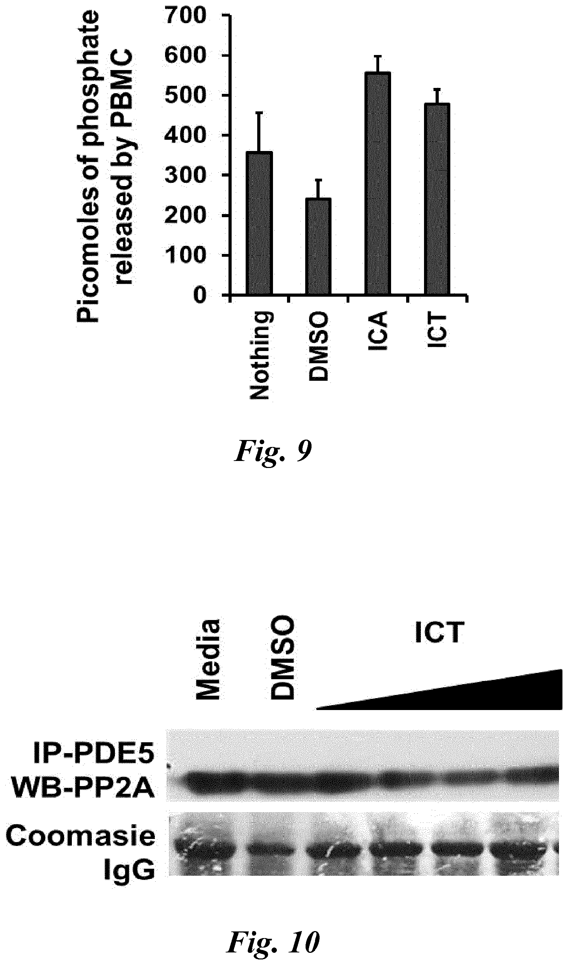

**Fig. 9** depicts the effect of ICA and ICT on the activity of PP2A. Human PBMC were cultured for 48 hours with media, DMSO, 1 µM of ICA or ICT. The cell lysates were used to analyze PP2A activity using a commercial *in vitro* phosphatase assay kit (Millipore). Error bars represent the SEM of three separate experiments.
**Fig. 10** depicts the binding of PP2A to PDE5. Human PBMC were cultured for 48 hours with media, DMSO or increasing doses of ICT (1, 5, 10, 20 µM respectively). Cells were then lysed and PDE5 was immunoprecipitated following a Western Blot with anti-PP2A. Coomasie blue staining of the membrane is shown as loading control.

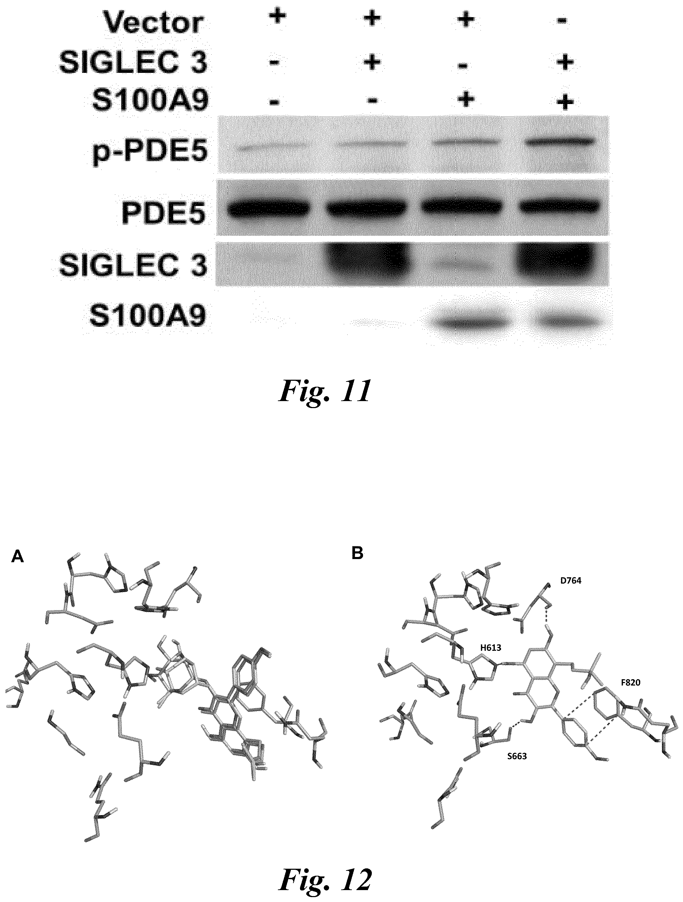

**Fig. 11** depicts the effect of S100A9/SIGLEC 3 signaling on the phosphorylation of PDE5. SJCRH30 transfected with either empty vector, SIGLEC 3 or S100A9 were cultured for 72 hours and assessed by Western Blot for either PDE5 or its activated counterpart ser92 of PDE5.
**Fig. 12** depicts the modeling results of Icarisid II self-docking and ICT docking. A) Model validation by docking Icarisid II and reproducing co-crystallized pose. Icarisid II docks into 2H44 with an RMS of 0.96 indicating that the model of 2H44 is robust and capable of predicting binding of Icarisid II and related compounds. B) Binding pose of ICT in 2H44. Interactions are indicated by blue dotted lines with hydrogen bonds predicted at and the potential pi stacking with F820 is indicated by the parallel blue dotted lines.

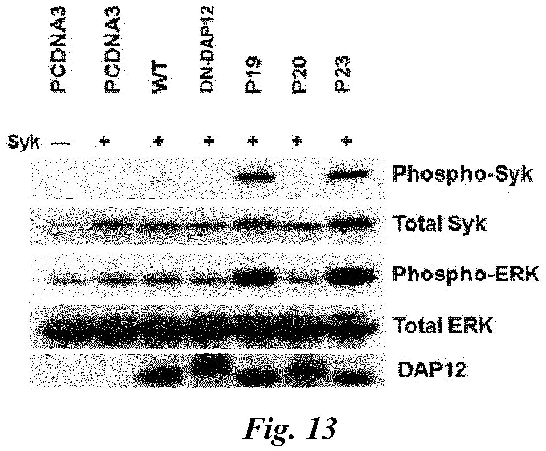

**Fig. 13** depicts the effect of active DAP12 on Syk and MAPK activation. AD293 cells were transfected with either vector alone or vector containing WT-DAP12, dominant negative DAP12 (DN) or active DAP12 (P19 and 23) for 48 hours. Cells lysates were prepared and Western blotting on whole cell lysate was performed. Total Syk and Total ERK were used as loading controls. This is representative of three independent experiments.

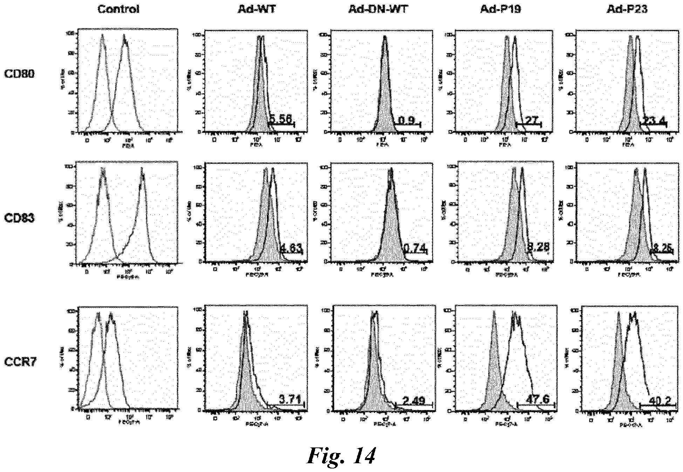

**Fig. 14** depicts the effect of active DAP12 on immature DC maturation. Primary DCs were prepared from healthy donors and infected with adenoviral vectors containing GFP alone, wt-DAP12, dominate negative DAP12 (dnDAP12) active DAP12 (ad-P19 and Ad-P23) as indicated. The cells were cultured for 72 hrs before flow cytometric analysis using mature DC surface marker as indicated. Mock infected DC and Isotype IgG included in control group. Each experimental construct was compared to the empty-vector control (filled histograms), and infected cells were gated on GFP prior to analysis.

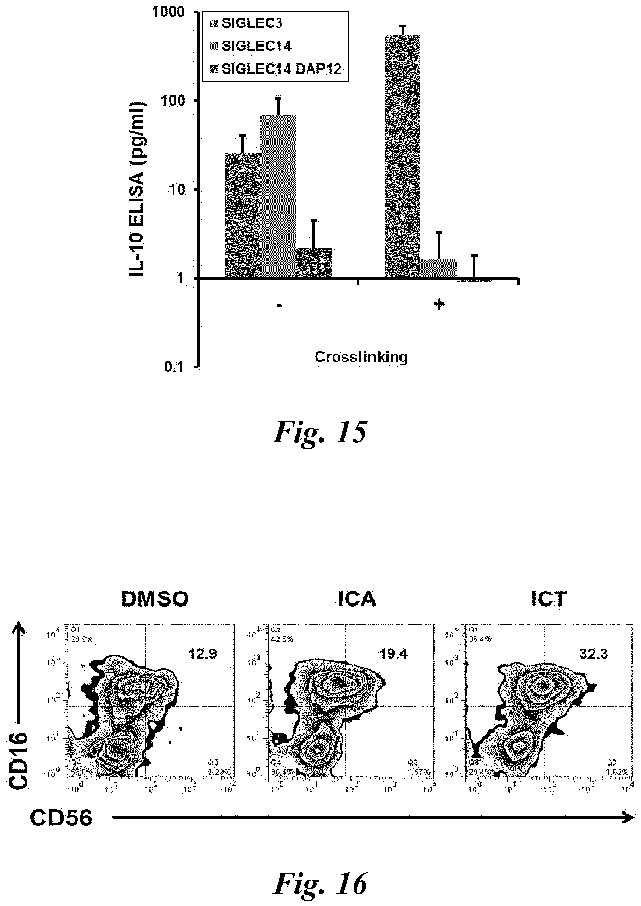

**Fig. 15** depicts the effect of SIGLEC14 and DAP12 on the secretion of IL-10. AD293 cells where either mock transfected or transfected with a control vector, SIGLEC14 plasmid or SIGLEC14 and wt-DAP12 followed by 72 hours of culture. The supernatants were collected and measured by ELISA as per the manufacturer's protocol. Bars represent the mean of three separate experiments as well as three separate measurements of each experiment. Error bars represent the SEM.
**Fig. 16** depicts the effect of ICA and ICT treatment on the surface expression of CD16 (FcγRIII) in NK cells. PBMC from healthy donors were treated with ICA/ICT or DMSO for 48 hours before analysis of CD16 expression by flow cytometry. NK cells were defined as CD3⁻ population and analyzed for the presence of CD16⁺CD56⁺ cells as indicated.

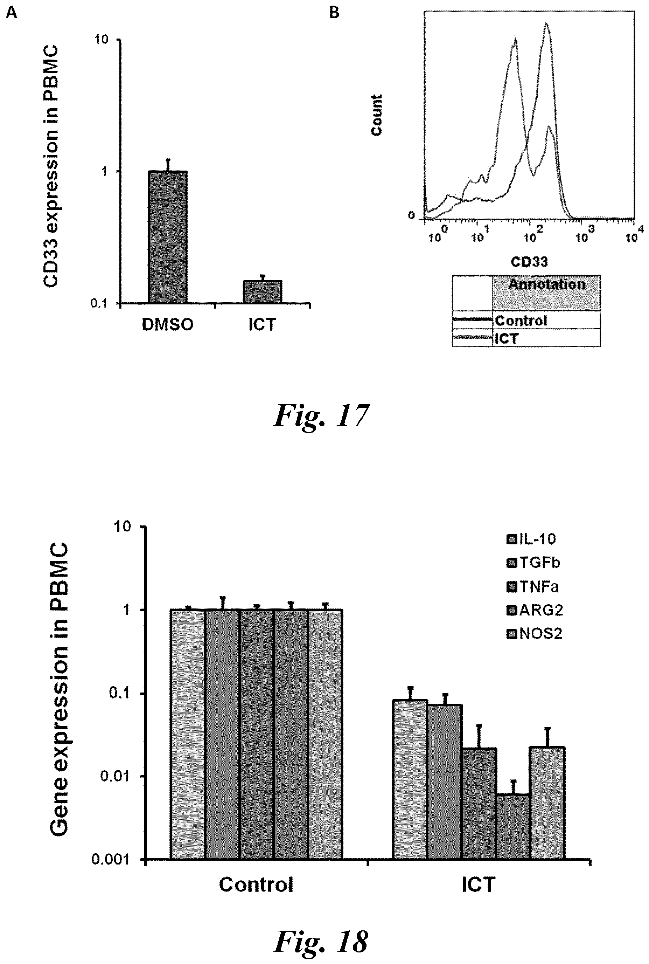

**Fig. 17A** and **17B** depict the effect of ICT on the expression of CD33 on PBMC.
**Fig. 18** depicts the effect of ICT on the expression of suppressive factors in PBMC.

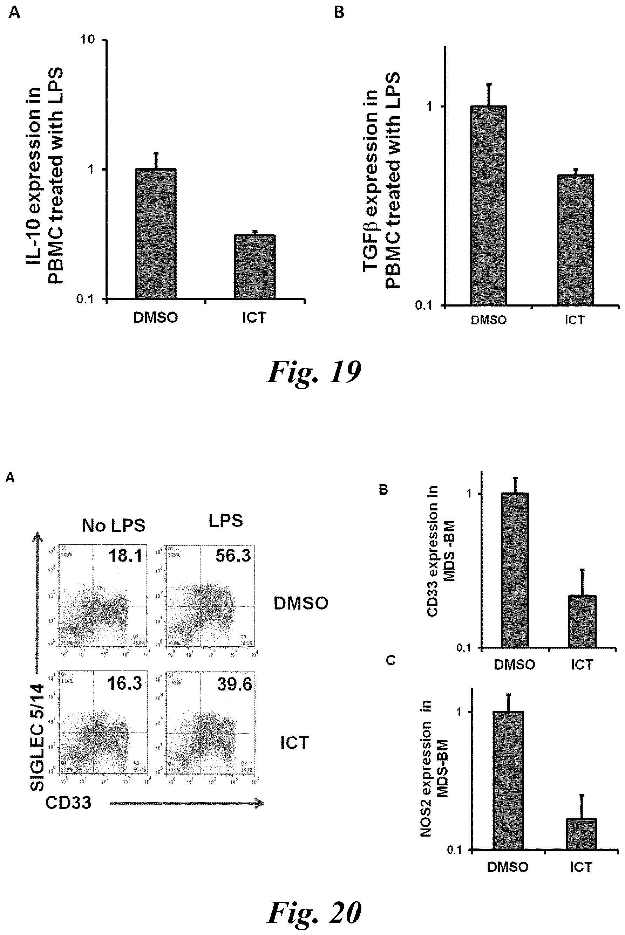

**Fig. 19A** and **19B** depict the effect of ICT on the expression of the suppressive cytokine IL-10 and TGFb on LPS-treated PBMC.
**Fig. 20A, 20B,** and **20C** depict the effect of ICT on the expression of CD33 and iNOS on MDS-BM.

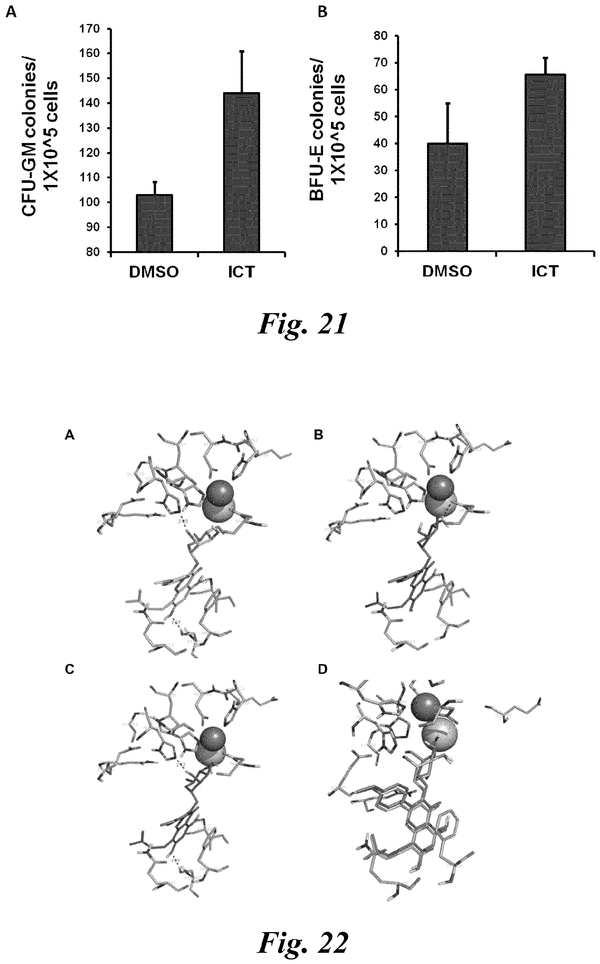

**Fig. 21A** and **21B** depict the effect of ICA and ICT on the hematopoeisis of MDS BMNCs.
**Fig. 22A, 22B, 22C** and **22D** depict the docking model of ICT in PDE5A1 *in silico.*

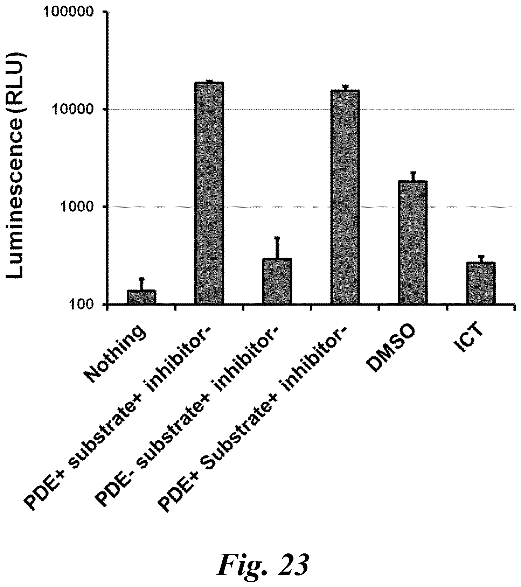

**Fig. 23** depicts the effect of ICA and ICT on the enzymatic activity of PDE5.

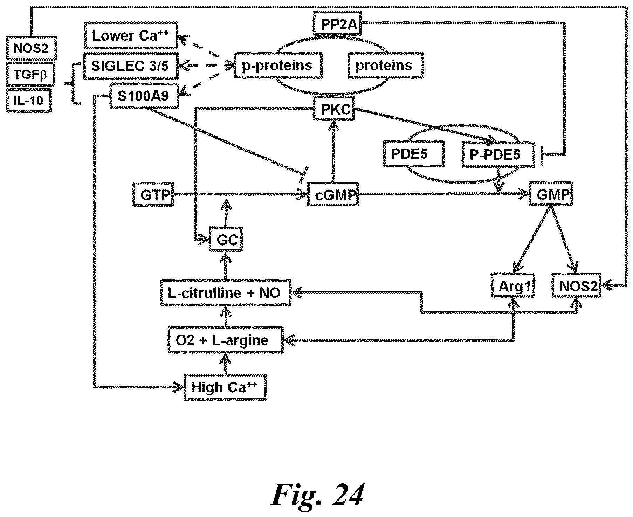

**Fig. 24** depicts a scheme of PDE5 signaling and its link to PP2A and S100A9.

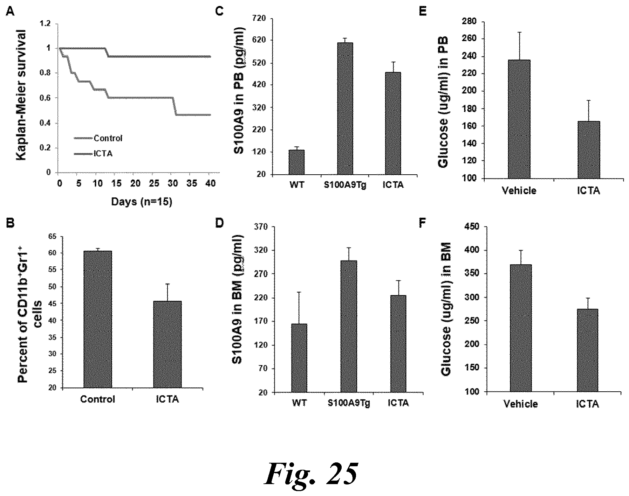

**Fig. 25** decicts the effect of ICT. A) Kaplan-Meier survival of aged S100A9Tg mice treated with either vehicle or ICT (50 µg/kg every other day for 40 days). B) Percent of CD11b+Gr1+ immature myeloid cells before and after treatment of the mice in A, measured by flow cytometry. Following these experiments, a long term treatment experiment starting at 3 month of age until 9 months of age in S100A9Tg mice was performed. The levels of circulating S100A9 were meadures by ELISA of C) peripheral blood (PB, obtained by heart puncture after euthanasia) and D) BM plasma (by removing the supernatant from BM collection and concentrating them to 500 µL in a 1K spin filter). The same plasma was used to measure glucose in E) PB and F) BM.

### DETAILED DESCRIPTION

The compounds, compositions and methods described herein may be understood more readily by reference to the following detailed description of specific aspects of the disclosed subject matter and the Examples and Figures included therein.

Before the present compounds, compositions and methods are disclosed and described, it is to be understood that the aspects described below are not limited to specific synthetic methods or specific reagents, as such may, of course, vary. It is also to be understood that the terminology used herein is for the purpose of describing particular aspects only and is not intended to be limiting.

Also, throughout this specification, various publications are referenced. The disclosures of these publications in their entireties are hereby incorporated by reference into this application in order to more fully describe the state of the art to which the disclosed matter pertains. The references disclosed are also individually and specifically incorporated by reference herein for the material contained in them that is discussed in the sentence in which the reference is relied upon.

### General Definitions

In this specification and in the claims that follow, reference will be made to a number of terms, which shall be defined to have the following meanings:
Throughout the description and claims of this specification the word "comprise" and other forms of the word, such as "comprising" and "comprises," means including but not limited to, and is not intended to exclude, for example, other additives, components, integers, or steps.
As used in the description and the appended claims, the singular forms "a," "an," and "the" include plural referents unless the context clearly dictates otherwise. Thus, for example, reference to "a composition" includes mixtures of two or more such compositions, reference to "an agent" includes mixtures of two or more such agents, reference to "the component" includes mixtures of two or more such components, and the like.
"Optional" or "optionally" means that the subsequently described event or circumstance can or cannot occur, and that the description includes instances where the event or circumstance occurs and instances where it does not.
Ranges can be expressed herein as from "about" one particular value, and/or to "about" another particular value. By "about" is meant within 5% of the value, e.g., within 4, 3, 2, or 1% of the value. When such a range is expressed, another aspect includes from the one particular value and/or to the other particular value. Similarly, when values are expressed as approximations, by use of the antecedent "about," it will be understood that the particular value forms another aspect. It will be further understood that the endpoints of each of the ranges are significant both in relation to the other endpoint, and independently of the other endpoint.
The term "inhibit" refers to a decrease in an activity, response, condition, disease, or other biological parameter. This can include but is not limited to the complete ablation of the activity, response, condition, or disease. This may also include, for example, a 10% reduction in the activity, response, condition, or disease as compared to the native or control level. Thus, the reduction can be a 10, 20, 30, 40, 50, 60, 70, 80, 90, 100%, or any amount of reduction in between as compared to native or control levels.
As used herein, by a "subject" is meant an individual. Thus, the "subject" can include domesticated animals (*e.g.,* cats, dogs, etc.), livestock (*e.g.,* cattle, horses, pigs, sheep, goats, etc.), laboratory animals (*e.g.,* mouse, rabbit, rat, guinea pig, etc.), and birds. "Subject" can also include a mammal, such as a primate or a human.
By "reduce" or other forms of the word, such as "reducing" or "reduction," is meant lowering of an event or characteristic (*e.g.,* tumor growth). It is understood that this is typically in relation to some standard or expected value, in other words it is relative, but that it is not always necessary for the standard or relative value to be referred to. For example, "reduces tumor growth" means reducing the rate of growth of a tumor relative to a standard or a control.
By "prevent" or other forms of the word, such as "preventing" or "prevention," is meant to stop a particular event or characteristic, to stabilize or delay the development or progression of a particular event or characteristic, or to minimize the chances that a particular event or characteristic will occur. Prevent does not require comparison to a control as it is typically more absolute than, for example, reduce. As used herein, something could be reduced but not prevented, but something that is reduced could also be prevented. Likewise, something could be prevented but not reduced, but something that is prevented could also be reduced. It is understood that where reduce or prevent are used, unless specifically indicated otherwise, the use of the other word is also expressly disclosed.
By "treat" or other forms of the word, such as "treated" or "treatment," is meant to administer a composition or to perform a method in order to reduce, prevent, inhibit, or eliminate a particular characteristic or event (*e.g.,* tumor growth or survival). The term "control" is used synonymously with the term "treat."
The term "anticancer" refers to the ability to treat or control cellular proliferation and/or tumor growth at any concentration.

### Chemical Definitions

As used herein, the term "substituted" is contemplated to include all permissible substituents of organic compounds. In a broad aspect, the permissible substituents include acyclic and cyclic, branched and unbranched, carbocyclic and heterocyclic, and aromatic and nonaromatic substituents of organic compounds. Illustrative substituents include, for example, those described below. The permissible substituents can be one or more and the same or different for appropriate organic compounds. For purposes of this disclosure, the heteroatoms, such as nitrogen, can have hydrogen substituents and/or any permissible substituents of organic compounds described herein which satisfy the valences of the heteroatoms. This disclosure is not intended to be limited in any manner by the permissible substituents of organic compounds. Also, the terms "substitution" or "substituted with" include the implicit proviso that such substitution is in accordance with permitted valence of the substituted atom and the substituent, and that the substitution results in a stable compound, *e.g.,* a compound that does not spontaneously undergo transformation such as by rearrangement, cyclization, elimination, etc.

"Z¹," "Z²," "Z³," and "Z⁴" are used herein as generic symbols to represent various specific substituents. These symbols can be any substituent, not limited to those disclosed herein, and when they are defined to be certain substituents in one instance, they can, in another instance, be defined as some other substituents.

The term "aliphatic" as used herein refers to a non-aromatic hydrocarbon group and includes branched and unbranched, alkyl, alkenyl, or alkynyl groups.

The term "alkyl" as used herein is a branched or unbranched saturated hydrocarbon group of 1 to 24 carbon atoms, for example 1 to 3, 1 to 4, 1 to 5, 1 to 6, 1 to 7, 1 to 8, 1 to 9, 1 to 10, or 1 to 15 carbon atoms, such as methyl, ethyl, n-propyl, isopropyl, n-butyl, isobutyl, t-butyl, pentyl, hexyl, heptyl, octyl, nonyl, decyl, dodecyl, tetradecyl, hexadecyl, eicosyl, tetracosyl, and the like. The alkyl group can also be substituted or unsubstituted. The alkyl group can be substituted with one or more groups including, but not limited to, alkyl, halogenated alkyl, alkoxy, alkenyl, alkynyl, aryl, heteroaryl, aldehyde, amino, carboxylic acid, ester, ether, halide, hydroxy, ketone, nitro, silyl, sulfo-oxo, sulfonyl, sulfone, sulfoxide, or thiol, as described below.

Throughout the specification "alkyl" is generally used to refer to both unsubstituted alkyl groups and substituted alkyl groups; however, substituted alkyl groups are also specifically referred to herein by identifying the specific substituent(s) on the alkyl group. For example, the term "halogenated alkyl" specifically refers to an alkyl group that is substituted with one or more halide, *e.g.,* fluorine, chlorine, bromine, or iodine. The term "alkoxyalkyl" specifically refers to an alkyl group that is substituted with one or more alkoxy groups, as described below. The term "alkylamino" specifically refers to an alkyl group that is substituted with one or more amino groups, as described below, and the like. When "alkyl" is used in one instance and a specific term such as "alkylalcohol" is used in another, it is not meant to imply that the term "alkyl" does not also refer to specific terms such as "alkylalcohol" and the like.

This practice is also used for other groups described herein. That is, while a term such as "cycloalkyl" refers to both unsubstituted and substituted cycloalkyl moieties, the substituted moieties can, in addition, be specifically identified herein; for example, a particular substituted cycloalkyl can be referred to as, *e.g.,* an "alkylcycloalkyl." Similarly, a substituted alkoxy can be specifically referred to as, *e.g.,* a "halogenated alkoxy," a particular substituted alkenyl can be, *e.g.,* an "alkenylalcohol," and the like. Again, the practice of using a general term, such as "cycloalkyl," and a specific term, such as "alkylcycloalkyl," is not meant to imply that the general term does not also include the specific term.

The term "alkoxy" as used herein is an alkyl group bound through a single, terminal ether linkage; that is, an "alkoxy" group can be defined as -OZ¹ where Z¹ is alkyl as defined above.

The term "alkenyl" as used herein is a hydrocarbon group of from 2 to 24 carbon atoms, for example, 2 to 5, 2 to 10, 2 to 15, or 2 to 20 carbon atoms, with a structural formula containing at least one carbon-carbon double bond. Asymmetric structures such as (Z¹Z²)C=C(Z³Z⁴) are intended to include both the *E* and *Z* isomers. This can be presumed in structural formulae herein wherein an asymmetric alkene is present, or it can be explicitly indicated by the bond symbol C=C. The alkenyl group can be substituted with one or more groups including, but not limited to, alkyl, halogenated alkyl, alkoxy, alkenyl, alkynyl, aryl, heteroaryl, aldehyde, amino, carboxylic acid, ester, ether, halide, hydroxy, ketone, nitro, silyl, sulfo-oxo, sulfonyl, sulfone, sulfoxide, or thiol, as described below.

The term "alkynyl" as used herein is a hydrocarbon group of 2 to 24 carbon atoms, for example 2 to 5, 2 to 10, 2 to 15, or 2 to 20 carbon atoms, with a structural formula containing at least one carbon-carbon triple bond. The alkynyl group can be substituted with one or more groups including, but not limited to, alkyl, halogenated alkyl, alkoxy, alkenyl, alkynyl, aryl, heteroaryl, aldehyde, amino, carboxylic acid, ester, ether, halide, hydroxy, ketone, nitro, silyl, sulfo-oxo, sulfonyl, sulfone, sulfoxide, or thiol, as described below.

The term "aryl" as used herein is a group that contains any carbon-based aromatic group including, but not limited to, benzene, naphthalene, phenyl, biphenyl, phenoxybenzene, and the like. The term "heteroaryl" is defined as a group that contains an aromatic group that has at least one heteroatom incorporated within the ring of the aromatic group. Examples of heteroatoms include, but are not limited to, nitrogen, oxygen, sulfur, and phosphorus. The term "non-heteroaryl," which is included in the term "aryl," defines a group that contains an aromatic group that does not contain a heteroatom. The aryl or heteroaryl group can be substituted or unsubstituted. The aryl or heteroaryl group can be substituted with one or more groups including, but not limited to, alkyl, halogenated alkyl, alkoxy, alkenyl, alkynyl, aryl, heteroaryl, aldehyde, amino, carboxylic acid, ester, ether, halide, hydroxy, ketone, nitro, silyl, sulfo-oxo, sulfonyl, sulfone, sulfoxide, or thiol as described herein. The term "biaryl" is a specific type of aryl group and is included in the definition of aryl. Biaryl refers to two aryl groups that are bound together *via* a fused ring structure, as in naphthalene, or are attached *via* one or more carbon-carbon bonds, as in biphenyl.

The term "cycloalkyl" as used herein is a non-aromatic carbon-based ring composed of at least three carbon atoms. Examples of cycloalkyl groups include, but are not limited to, cyclopropyl, cyclobutyl, cyclopentyl, cyclohexyl, etc. The term "heterocycloalkyl" is a cycloalkyl group as defined above where at least one of the carbon atoms of the ring is substituted with a heteroatom such as, but not limited to, nitrogen, oxygen, sulfur, or phosphorus. The cycloalkyl group and heterocycloalkyl group can be substituted or unsubstituted. The cycloalkyl group and heterocycloalkyl group can be substituted with one or more groups including, but not limited to, alkyl, alkoxy, alkenyl, alkynyl, aryl, heteroaryl, aldehyde, amino, carboxylic acid, ester, ether, halide, hydroxy, ketone, nitro, silyl, sulfo-oxo, sulfonyl, sulfone, sulfoxide, or thiol as described herein.

The term "cycloalkenyl" as used herein is a non-aromatic carbon-based ring composed of at least three carbon atoms and containing at least one double bound, *i.e.,* C=C. Examples of cycloalkenyl groups include, but are not limited to, cyclopropenyl, cyclobutenyl, cyclopentenyl, cyclopentadienyl, cyclohexenyl, cyclohexadienyl, and the like. The term "heterocycloalkenyl" is a type of cycloalkenyl group as defined above, and is included within the meaning of the term "cycloalkenyl," where at least one of the carbon atoms of the ring is substituted with a heteroatom such as, but not limited to, nitrogen, oxygen, sulfur, or phosphorus. The cycloalkenyl group and heterocycloalkenyl group can be substituted or unsubstituted. The cycloalkenyl group and heterocycloalkenyl group can be substituted with one or more groups including, but not limited to, alkyl, alkoxy, alkenyl, alkynyl, aryl, heteroaryl, aldehyde, amino, carboxylic acid, ester, ether, halide, hydroxy, ketone, nitro, silyl, sulfo-oxo, sulfonyl, sulfone, sulfoxide, or thiol as described herein.

The term "cyclic group" is used herein to refer to either aryl groups, non-aryl groups (*i.e.,* cycloalkyl, heterocycloalkyl, cycloalkenyl, and heterocycloalkenyl groups), or both. Cyclic groups have one or more ring systems that can be substituted or unsubstituted. A cyclic group can contain one or more aryl groups, one or more non-aryl groups, or one or more aryl groups and one or more non-aryl groups.

The term "carbonyl as used herein is represented by the formula -C(O)Z¹ where Z¹ can be a hydrogen, hydroxyl, alkoxy, alkyl, halogenated alkyl, alkenyl, alkynyl, aryl, heteroaryl, cycloalkyl, cycloalkenyl, heterocycloalkyl, or heterocycloalkenyl group described above. Throughout this specification "C(O)" or "CO" is a short hand notation for C=O.

The term "aldehyde" as used herein is represented by the formula -C(O)H.

The terms "amine" or "amino" as used herein are represented by the formula-NZ¹Z², where Z¹ and Z² can each be substitution group as described herein, such as hydrogen, an alkyl, halogenated alkyl, alkenyl, alkynyl, aryl, heteroaryl, cycloalkyl, cycloalkenyl, heterocycloalkyl, or heterocycloalkenyl group described above. "Amido" is -C(O)NZ¹Z².

The term "carboxylic acid" as used herein is represented by the formula -C(O)OH. A "carboxylate" or "carboxyl" group as used herein is represented by the formula -C(O)O⁻.

The term "ester" as used herein is represented by the formula -OC(O)Z¹ or -C(O)OZ¹, where Z¹ can be an alkyl, halogenated alkyl, alkenyl, alkynyl, aryl, heteroaryl, cycloalkyl, cycloalkenyl, heterocycloalkyl, or heterocycloalkenyl group described above.

The term "ether" as used herein is represented by the formula Z¹OZ², where Z¹ and Z² can be, independently, an alkyl, halogenated alkyl, alkenyl, alkynyl, aryl, heteroaryl, cycloalkyl, cycloalkenyl, heterocycloalkyl, or heterocycloalkenyl group described above.

The term "ketone" as used herein is represented by the formula Z¹C(O)Z², where Z¹ and Z² can be, independently, an alkyl, halogenated alkyl, alkenyl, alkynyl, aryl, heteroaryl, cycloalkyl, cycloalkenyl, heterocycloalkyl, or heterocycloalkenyl group described above.

The term "halide" or "halogen" as used herein refers to the fluorine, chlorine, bromine, and iodine.

The term "hydroxyl" as used herein is represented by the formula -OH.

The term "nitro" as used herein is represented by the formula -NO₂.

The term "silyl" as used herein is represented by the formula -SiZ¹Z²Z³, where Z¹, Z², and Z³ can be, independently, hydrogen, alkyl, halogenated alkyl, alkoxy, alkenyl, alkynyl, aryl, heteroaryl, cycloalkyl, cycloalkenyl, heterocycloalkyl, or heterocycloalkenyl group described above.

The term "sulfonyl" is used herein to refer to the sulfo-oxo group represented by the formula -S(O)₂Z¹, where Z¹ can be hydrogen, an alkyl, halogenated alkyl, alkenyl, alkynyl, aryl, heteroaryl, cycloalkyl, cycloalkenyl, heterocycloalkyl, or heterocycloalkenyl group described above.

The term "sulfonylamino" or "sulfonamide" as used herein is represented by the formula -S(O)₂NH-.

The term "thiol" as used herein is represented by the formula -SH.

The term "thio" as used herein is represented by the formula -S-.

"R¹," "R²," "R³," "Rⁿ," etc., where n is some integer, as used herein can, independently, possess one or more of the groups listed above. For example, if R¹ is a straight chain alkyl group, one of the hydrogen atoms of the alkyl group can optionally be substituted with a hydroxyl group, an alkoxy group, an amine group, an alkyl group, a halide, and the like. Depending upon the groups that are selected, a first group can be incorporated within second group or, alternatively, the first group can be pendant (i.e., attached) to the second group. For example, with the phrase "an alkyl group comprising an amino group," the amino group can be incorporated within the backbone of the alkyl group. Alternatively, the amino group can be attached to the backbone of the alkyl group. The nature of the group(s) that is (are) selected will determine if the first group is embedded or attached to the second group.

Unless stated to the contrary, a formula with chemical bonds shown only as solid lines and not as wedges or dashed lines contemplates each possible isomer, *e.g.,* each enantiomer, diastereomer, and meso compound, and a mixture of isomers, such as a racemic or scalemic mixture.

Reference will now be made in detail to specific aspects of the disclosed materials, compounds, compositions, articles, and methods, examples of which are illustrated in the accompanying Examples and Figures.

### Compounds

Icariin (ICA) is a flavonoid glycoside derived from epimedium plants. Epimedium plants, also known as horny goat weed in the west or as Yinyanghuo in the Chinese pharmacopeia, contain an abundance of flavonoid glycosides. Icariin and its deglycosolated derivative icaritin (3,5,7-trihydroxy-2-(4-methoxyphenyl)-8-(3-methyl-2-buten-1-yl)-4H-1-benzopyran-4-one), are thought to be responsible for the effects observed from herbal extracts of these plants, including enhanced anti-inflammatory and anti-tumorigenic activities.

ICA and a derivative 3,5,7-trihydroxy-4'-methoxy-8-(3-hydroxy-3-methylbutyl)-flavone) (ICT), were recently identified to effectively inhibit inflammatory responses associated with MDSCs (Zhou J et al. Int Immunopharmacol. 2011;11(7):890-8; Wu J et al. Int Immunopharmacol. 2011;12(1):74-9, which are incorporated by reference herein in their entirities for their teachings of ICA and ICT and their effect and use on MDSC and cancers). These compounds disrupt the interaction of S100A8/A9 by reducing their expression, leading to a decrease in the number of peripheral and intratumoral MDSCs and inactivation of their activity, resulting in a reduced tumor burden. Thus, disclosed herein in one aspect are pharmaceutical compositions comprising ICA, icaritin, and/or ICT with a pharmaceutical carrier, and optional anti-cancer and/or anti-inflammatory agent. Also, disclosed herein in one aspect are pharmaceutical compositions comprising extracts of Epimedium plants with a pharmaceutical carrier, and optional anti-cancer and/or anti-inflammatory agent.

In a further aspect, disclosed herein are compounds that are derivatives of ICA and/or ICT. For example, disclosed herein are compounds having Formula I: 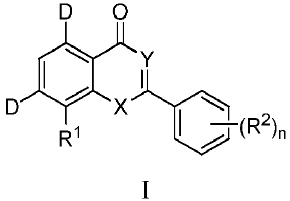 wherein,
Y is chosen from N, COH, COR, and CR¹;
X is chosen from NH and O, with the proviso that when Y is N, X is NH and when Y is COH, COR, or CR¹, X is O;
each D, independent of the other, is chosen from H, OH, OR, and halogen; R is alkyl or monoglucoside;
R¹ is chosen from hydrogen, halogen, hydroxyl, amino, thiol, thioalkyl, alkyl, alkenyl, alkynyl, haloalkyl, cycloalkyl, heterocycloalkyl, alkylaryl, aryl, alkylheteroaryl, or heteroaryl, any of which is optionally substituted with acetyl, alkyl, amino, amido, alkoxyl, alkylhydroxy, cycloalkyl, heterocycloalkyl, aryl, heteroaryl, carbonyl, halogen, hydroxyl, thiol, cyano, or nitro;
each R², independent of any other, is chosen from hydrogen, hydroxyl, alkoxyl, sulfonyl, amino, thiol, thioalkyl, alkyl, alkenyl, alkynyl, haloalkyl, cycloalkyl, heterocycloalkyl, alkylaryl, aryl, alkylheteroaryl, or heteroaryl, any of which is optionally substituted with acetyl, alkyl, amino, amido, alkoxy, alkylhydroxy, cycloalkyl, heterocycloalkyl, aryl, heteroaryl, carbonyl, halogen, hydroxyl, thiol, cyano, nitro, sulfonyl, or sulfonlylamino;
n is 0, 1, 2, 3, 4 or 5;
or a pharmaceutically acceptable salt or prodrug thereof.

In certain examples, each D, independent of the other, is selected from H, OH, OR, and halogen. In other specific examples, one D is H. In other examples, both D are H. In still other examples, one D is OH. In other examples, both D are OH. In yet further examples, one D is OR. In still other examples, both D are OR.

In certain examples, R¹ is alkyl, alkenyl, or alkoxyl, optionally substituted with with acetyl, alkyl, amino, amido, alkoxy, alkylhydroxy, cycloalkyl, heterocycloalkyl, aryl, heteroaryl, carbonyl, halogen, or hydroxyl. The alkyl or alkenyl can be from C₁ to C₂₄, more specifically, from C₁ to C₁₂, more specifically, from C₁ to C₈, such as from C₃ to C₆ in length.

In certain examples, R² is alkyl, alkenyl, or alkoxyl, optionally substituted with with acetyl, alkyl, amino, amido, alkoxy, alkylhydroxy, cycloalkyl, heterocycloalkyl, aryl, heteroaryl, carbonyl, halogen, hydroxyl, sulfonyl, or sulfonlylamino. For example, R² can be methoxyl, ethoxyl, propyloxyl, methyl, ethyl, or propyl.

In certain examples, the disclosed compounds not include icariin or icaritin. Thus, compounds of Formula I can include the proviso that X is not O, Y is not COH or COR, each D are not both OH or both OR, where R is a monosaccharide, R¹ is not 3-methyl-2-butenyl, and R² is not n-methoxy. However, compositions containing icariin and icaratin with pharmaceutical carriers and optional anticancer or antiinflammation agents are disclosed herein.

In some specific examples of Formula I, where Y is COH, and X is O, the compounds have Formula I-A: 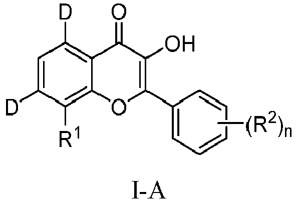 where D, R¹, n, and R² are as defined herein.

In some specific examples of Formula I, where Y is COH, and X is O, the compounds have Formula I-B: 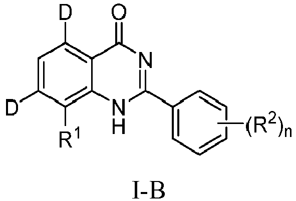 where D, R¹, n, and R² are as defined herein.

In some specific examples of Formula I, where each D is OH, Y is COH, X is O, n is 1, R² is methoxyl, and R¹ is CH₂CH₂R³, the compounds have Formula II: 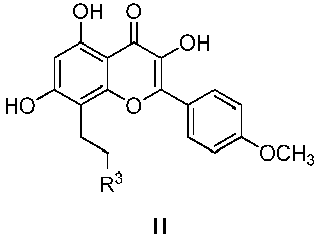
wherein R³ is selected from hydrogen, halogen, hydroxyl, amino, alkyl, alkenyl, alkynyl, haloalkyl, cycloalkyl, heterocycloalkyl, alkylaryl, aryl, alkylheteroaryl, or heteroaryl, any of which is optionally substituted with carbonyl, alkyl, amino, amido, alkoxyl, alkylhydroxy, cycloalkyl, heterocycloalkyl, aryl, heteroaryl, carbonyl, halogen, hydroxyl, thiol, cyano, or nitro;
with the proviso that R³ is not chloroisopropyl, hydroxyisopropyl, isopropylacetamide, aminoisopropyl, methoxyisopropyl;
or a pharmaceutically acceptable salt or prodrug thereof. The proviso of icariin and icaritin noted above can also apply to Formula II.

In some examples of Formula II, R³ is an alkenyl group, optionally substituted with carbonyl, alkyl, amino, amido, alkoxyl, alkylhydroxy, cycloalkyl, heterocycloalkyl, aryl, heteroaryl, carbonyl, halogen, hydroxyl, thiol, cyano, or nitro. In other examples, R³ is an alkyl group optionally substituted with carbonyl, alkyl, amino, amido, alkoxyl, alkylhydroxy, cycloalkyl, heterocycloalkyl, aryl, heteroaryl, carbonyl, halogen, hydroxyl, thiol, cyano, or nitro.

In one specific example, the compound is ICT

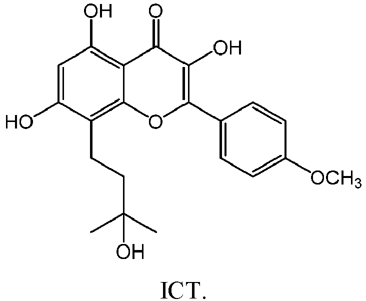

In some further examples, where R³ is NHR⁴, compounds are of Formula III: 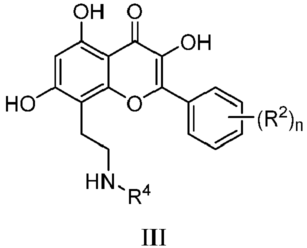
wherein R⁴ is selected from alkyl, alkenyl, alkynyl, haloalkyl, cycloalkyl, heterocycloalkyl, alkylaryl, aryl, alkylheteroaryl, or heteroaryl, any of which is optionally substituted with carbonyl, alkyl, amino, amido, alkoxyl, alkylhydroxy, cycloalkyl, heterocycloalkyl, aryl, heteroaryl, carbonyl, halogen, hydroxyl, thiol, cyano, or nitro;
each R², independent of any other, is chosen from hydrogen, hydroxyl, alkoxyl, sulfonyl, amino, thiol, thioalkyl, alkyl, alkenyl, alkynyl, haloalkyl, cycloalkyl, heterocycloalkyl, alkylaryl, aryl, alkylheteroaryl, or heteroaryl, any of which is optionally substituted with acetyl, alkyl, amino, amido, alkoxy, alkylhydroxy, cycloalkyl, heterocycloalkyl, aryl, heteroaryl, carbonyl, halogen, hydroxyl, thiol, cyano, nitro, sulfonyl, or sulfonlylamino;
n is 0, 1, 2, 3, 4 or 5;
or a pharmaceutically acceptable salt or prodrug thereof.

A further example of compounds of Formula III, is where R² is para methoxy, which is represented by Formula III-A:

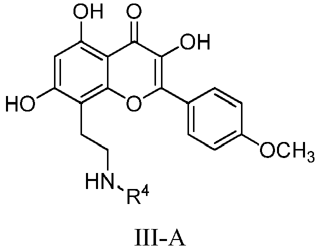

In some specific examples of Formula III, where R⁴ is C(O)R⁵, compounds are of Formula IV: 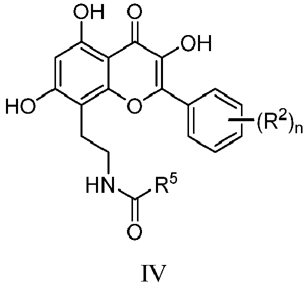
wherein R⁵ is selected from alkyl, alkenyl, alkynyl, haloalkyl, cycloalkyl, heterocycloalkyl, alkylaryl, aryl, alkylheteroaryl, or heteroaryl, any of which is optionally substituted with carbonyl, alkyl, amino, amido, alkoxyl, alkylhydroxy, cycloalkyl, heterocycloalkyl, aryl, heteroaryl, carbonyl, halogen, hydroxyl, thiol, cyano, or nitro;
each R², independent of any other, is chosen from hydrogen, hydroxyl, alkoxyl, sulfonyl, amino, thiol, thioalkyl, alkyl, alkenyl, alkynyl, haloalkyl, cycloalkyl, heterocycloalkyl, alkylaryl, aryl, alkylheteroaryl, or heteroaryl, any of which is optionally substituted with acetyl, alkyl, amino, amido, alkoxy, alkylhydroxy, cycloalkyl, heterocycloalkyl, aryl, heteroaryl, carbonyl, halogen, hydroxyl, thiol, cyano, nitro, sulfonyl, or sulfonlylamino;
n is 0, 1, 2, 3, 4 or 5;
or a pharmaceutically acceptable salt or prodrug thereof.

A further example of compounds of Formula IV, is where R² is para methoxy, which is represented by Formula IV-A:

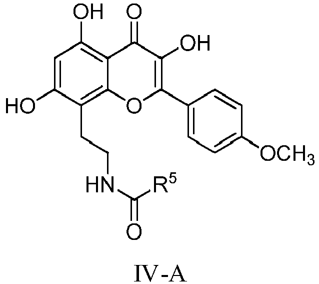

In some further embodiments of Formula I, where each D is OH, Y is COH, X is O, n is 1, R² is methoxyl, and R¹ is CH₂C(O)NR⁶R⁷, compounds are of Formula V: 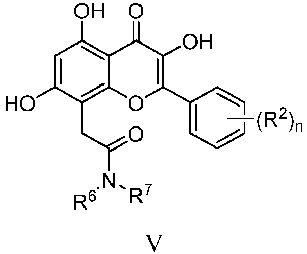
wherein R⁶ and R⁷ are independently selected from alkyl, alkenyl, alkynyl, haloalkyl, cycloalkyl, heterocycloalkyl, alkylaryl, aryl, alkylheteroaryl, or heteroaryl, any of which is optionally substituted with carbonyl, alkyl, amino, amido, alkoxyl, alkylhydroxy, cycloalkyl, heterocycloalkyl, aryl, heteroaryl, carbonyl, halogen, hydroxyl, thiol, cyano, or nitro;
each R², independent of any other, is chosen from hydrogen, hydroxyl, alkoxyl, sulfonyl, amino, thiol, thioalkyl, alkyl, alkenyl, alkynyl, haloalkyl, cycloalkyl, heterocycloalkyl, alkylaryl, aryl, alkylheteroaryl, or heteroaryl, any of which is optionally substituted with acetyl, alkyl, amino, amido, alkoxy, alkylhydroxy, cycloalkyl, heterocycloalkyl, aryl, heteroaryl, carbonyl, halogen, hydroxyl, thiol, cyano, nitro, sulfonyl, or sulfonlylamino;
n is 0, 1, 2, 3, 4 or 5;
or a pharmaceutically acceptable salt or prodrug thereof.

A further example of compounds of Formula V, is where R² is para methoxy, which is represented by Formula V-A:

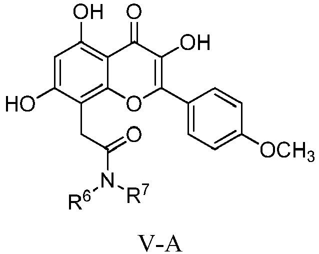

In some embodiments of Formula I, where each D is OH, Y is COH, X is O, n is 1, R² is methoxyl, and R¹ is CH₂CH=CR⁸R⁹, compounds are of Formula VI: 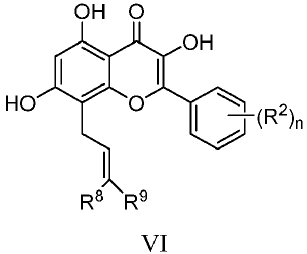
wherein R⁸ and R⁹ are independently selected from alkyl, haloalkyl, cycloalkyl, heterocycloalkyl, alkylaryl, aryl, alkylheteroaryl, or heteroaryl, any of which is optionally substituted with carbonyl, alkyl, amino, amido, alkoxyl, alkylhydroxy, cycloalkyl, heterocycloalkyl, aryl, heteroaryl, carbonyl, halogen, hydroxyl, thiol, cyano, or nitro;
with the proviso that R⁸ and R⁹ are not both be methyl;
each R², independent of any other, is chosen from hydrogen, hydroxyl, alkoxyl, sulfonyl, amino, thiol, thioalkyl, alkyl, alkenyl, alkynyl, haloalkyl, cycloalkyl, heterocycloalkyl, alkylaryl, aryl, alkylheteroaryl, or heteroaryl, any of which is optionally substituted with acetyl, alkyl, amino, amido, alkoxy, alkylhydroxy, cycloalkyl, heterocycloalkyl, aryl, heteroaryl, carbonyl, halogen, hydroxyl, thiol, cyano, nitro, sulfonyl, or sulfonlylamino;
n is 0, 1, 2, 3, 4 or 5;
or a pharmaceutically acceptable salt or prodrug thereof.

A further example of compounds of Formula VI, is where R² is para methoxy, which is represented by Formula VI-A:

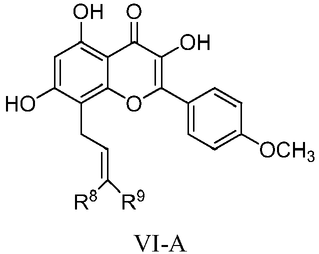

In some embodiments of Formlula I, where each D is OH, Y is N, X is NH, n is 2, one R² is OCH₂CH₃, and the other R² is S(O)(O)NR⁶R⁷, compounds are of Formula VII: 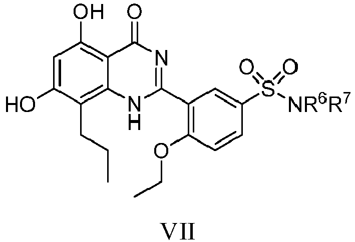 wherein R⁶ and R⁷ are independently selected from alkyl, alkenyl, alkynyl, haloalkyl, cycloalkyl, heterocycloalkyl, alkylaryl, aryl, alkylheteroaryl, or heteroaryl, any of which is optionally substituted with carbonyl, alkyl, amino, amido, alkoxyl, alkylhydroxyl, cycloalkyl, heterocycloalkyl, aryl, heteroaryl, carbonyl, halogen, hydroxyl, thiol, cyano, or nitro;
or a pharmaceutically acceptable salt or prodrug thereof.

In some embodiments of Formula I, where each D is H, Y is N, X is NH, n is 2, one R² is OCH₂CH₃, and the other R² is S(O)(O)NR⁶R⁷, compounds are of Formula VIII: 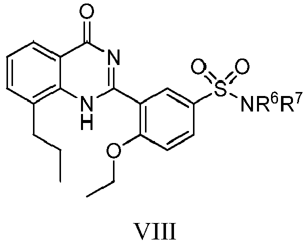 wherein R⁶ and R⁷ are independently selected from alkyl, alkenyl, alkynyl, haloalkyl, cycloalkyl, heterocycloalkyl, alkylaryl, aryl, alkylheteroaryl, or heteroaryl, any of which is optionally substituted with carbonyl, alkyl, amino, amido, alkoxyl, alkylhydroxyl, cycloalkyl, heterocycloalkyl, aryl, heteroaryl, carbonyl, halogen, hydroxyl, thiol, cyano, or nitro;
or a pharmaceutically acceptable salt or prodrug thereof.

In some embodiments of Formula I, each D is H, Y is COH, X is O, n is 2, one R² is OCH₂CH₃, and the other R² is S(O)(O)NR⁶R⁷, where R⁶ and R⁷ are as defined herein.

Also disclosed herein are pharmaceutically-acceptable salts and prodrugs of the disclosed compounds. Pharmaceutically-acceptable salts include salts of the disclosed compounds that are prepared with acids or bases, depending on the particular substituents found on the compounds. Under conditions where the compounds disclosed herein are sufficiently basic or acidic to form stable nontoxic acid or base salts, administration of the compounds as salts can be appropriate. Examples of pharmaceutically-acceptable base addition salts include sodium, potassium, calcium, ammonium, or magnesium salt. Examples of physiologically-acceptable acid addition salts include hydrochloric, hydrobromic, nitric, phosphoric, carbonic, sulphuric, and organic acids like acetic, propionic, benzoic, succinic, fumaric, mandelic, oxalic, citric, tartaric, malonic, ascorbic, alpha-ketoglutaric, alpha-glycophosphoric, maleic, tosyl acid, methanesulfonic, and the like. Thus, disclosed herein are the hydrochloride, nitrate, phosphate, carbonate, bicarbonate, sulfate, acetate, propionate, benzoate, succinate, fumarate, mandelate, oxalate, citrate, tartarate, malonate, ascorbate, alpha-ketoglutarate, alpha-glycophosphate, maleate, tosylate, and mesylate salts. Pharmaceutically acceptable salts of a compound can be obtained using standard procedures well known in the art, for example, by reacting a sufficiently basic compound such as an amine with a suitable acid affording a physiologically acceptable anion. Alkali metal (for example, sodium, potassium or lithium) or alkaline earth metal (for example calcium) salts of carboxylic acids can also be made.

Compounds of Formulas I-VIII can be prepared beginning from Icaritin. For example the isopreneyl moiety on Icaritin can be oxidixed to an aldehyde, which can be reductively aminated to the amine or amide, or to the ester, which can be converted into the amide. Still further, the isoprenyl moiety can be oxidized to a carbonyl, which can be converted into a suitable leaving group for substitution reactions.

### Methods of Use

ICA, icariti, ICT and their derivatives disclosed herein can be used to modulate the activation of MDSCs and alter the tumor microenvironment created by MDSCs. These compounds, and compositions containing them, can act through the down-regulation of S100A9/SIGLEC3 signaling, which is primordial to the function of MDSCs. The signaling event targeted by ICA/ICT and their derivatives disclosed herein can include direct or indirect inhibition of PDE5 and the activation of PP2A, which controls inflammatory mediators including the NO produced by MDSC. ICA/ICT and their derivatives disclosed herein can also be used to activate DAP12 to inhibit SIGLEC3-ITIM signaling and reduce the number of MDSC by driving their maturation. Treatment with ICA/ICT and its derivatives disclosed herein can reduce TNFα which mediates NO production. ICA/ICT and its derivatives disclosed herein can also down-regulate the levels of STAT3, which is a well-established transcription factor for MDSC expansion as well as production of suppressive cytokines (e.g. TGFβ), angiogenic factors (VEGF) and survival factors that benefit the establishment of the tumor. The receptor/ligand interactions that trigger these pathways are unclear but TLR4 has been favored as a major trigger in MDSC development leading to inflammation and cancer. TLR4 is a specialized receptor that can recognize not only exogenous but also endogenous danger signals, comprising pathogen-associated molecular patterns (PAMPs) as well as endogenous danger signals (DAMPs). S100A8/A9 is a potent DAMP released by cells that activate TLR4. The TLR4/MyD88/IRAK pathway can be critical for activation of numerous downstream effector pathways, including NF-κB, MAPK and STAT3. Deficiency of any of these markers can be associated with reduced tumor growth. It has been suggested that one of the most important tumor-promoting properties of these DAMP/receptor interactions is their ability to recruit MDSC to the tumor site. Therefore, in the context of cancer in a sterile environment, DAMPs can be responsible for setting off an inflammatory response that promotes tumor progression and local immune suppression.

Disclosed herein are thus methods of treating or preventing cancer in a subject, comprising administering to the subject an effective amount of a compound or composition as disclosed herein. Further provided herein are methods of treating a precancerous syndrome in a subject, comprising administering to the subject an effective amount of a compound or composition as disclosed herein. Examples of a precancerous syndromes, include, but are not limited to, myelodysplastic syndrome, essential throbocythaemia, myelofibrosis, monoclonal gammopathy of unknown significance (MGUS), polycythaemia vera, adenomatous polyps, familial adenomatous polyposis, hereditaty non-polyposis colon cancer, submucous fibrosis, lichen planus, epidermolysis bullosa, discoid lupus erythematous, cervical dysplasia, cervical intraepithelial neoplasia, squamous intraepithelial lesion, epithelial hyperplasias, ductal carcinoma, and Paget's disease. Also provided are methods of sensitizing tumors to standard care therapy, comprising administering to the subject an effective amount of a compound or composition as disclosed herein.

Methods of killing a tumor cell are also provided herein. The methods comprise contacting a tumor cell with an effective amount of a compound or composition as disclosed herein. The methods can further include administering a second compound or composition (*e.g.,* an anticancer agent) or administering an effective amount of ionizing radiation to the subj ect.

Methods of modifying a tumor microenvironment are also provided herein. The methods comprise contacting a tumor with an effective amount of a compound or composition as disclosed herein. Modification of the microenvironment can be characterized by a reduction in MDSCs as compared to control. The methods can further include administering a second compound or composition (*e.g.,* an anticancer agent) or administering an effective amount of ionizing radiation to the subject.

Also provided herein are methods of radiotherapy of tumors, comprising contacting the tumor with an effective amount of a compound or composition as disclosed herein and irradiating the tumor with an effective amount of ionizing radiation. Methods of treating inflammation in a subject are further provided herein, the methods comprising administering to the subject an effective amount of a compound or composition as described herein. Optionally, the methods can further include administering a second compound or composition (*e.g.,* an anti-inflammatory agent).

The disclosed subject matter also concerns methods for treating a subject having an oncological disorder or condition. In one embodiment, an effective amount of one or more compounds or compositions disclosed herein is administered to a subject having an oncological disorder and who is in need of treatment thereof. The disclosed methods can optionally include identifying a subject who is or can be in need of treatment of an oncological disorder. The subject can be a human or other mammal, such as a primate (monkey, chimpanzee, ape, *etc*.), dog, cat, cow, pig, horse, mouse or other animals having an oncological disorder. Means for administering and formulating compounds for administration to a subject are known in the art, examples of which are described herein. Oncological disorders include, but are not limited to, precancerous syndromes (such as MDS), cancer and/or tumors of the anus, bile duct, bladder, bone, bone marrow, bowel (including colon and rectum), breast, eye, gall bladder, kidney, mouth, larynx, esophagus, stomach, testis, cervix, head, neck, ovary, lung, mesothelioma, neuroendocrine, penis, skin, spinal cord, thyroid, vagina, vulva, uterus, liver, muscle, pancreas, prostate, blood cells (including lymphocytes and other immune system cells), and brain. Specific cancers contemplated for treatment include B cell cancers such as leukemia (acute lymphoblastic, acute myeloid, chronic lymphocytic, chronic myeloid, and other), lymphoma (Hodgkin's and non-Hodgkin's), and multiple myeloma.

Other examples of cancers that can be treated according to the methods disclosed herein are adrenocortical carcinoma, adrenocortical carcinoma, cerebellar astrocytoma, basal cell carcinoma, bile duct cancer, bladder cancer, bone cancer, brain tumor, breast cancer, Burkitt's lymphoma, carcinoid tumor, central nervous system lymphoma, cervical cancer, chronic myeloproliferative disorders, colon cancer, cutaneous T-cell lymphoma, endometrial cancer, ependymoma, esophageal cancer, gallbladder cancer, gastric (stomach) cancer, gastrointestinal carcinoid tumor, germ cell tumor, glioma,, hairy cell leukemia, head and neck cancer, hepatocellular (liver) cancer, hypopharyngeal cancer, hypothalamic and visual pathway glioma, intraocular melanoma, retinoblastoma, islet cell carcinoma (endocrine pancreas), laryngeal cancer, lip and oral cavity cancer, liver cancer, medulloblastoma, Merkel cell carcinoma, squamous neck cancer with occult mycosis fungoides, myelodysplastic syndromes, myelogenous leukemia, nasal cavity and paranasal sinus cancer, nasopharyngeal cancer, neuroblastoma, non-small cell lung cancer, oral cancer, oropharyngeal cancer, osteosarcoma, ovarian cancer, pancreatic cancer, paranasal sinus and nasal cavity cancer, parathyroid cancer, penile cancer, pheochromocytoma, pineoblastoma and supratentorial primitive neuroectodermal tumor, pituitary tumor, plasma cell neoplasm/multiple myeloma, pleuropulmonary blastoma, prostate cancer, rectal cancer, renal cell (kidney) cancer, retinoblastoma, rhabdomyosarcoma, salivary gland cancer, Ewing's sarcoma, soft tissue sarcoma, Sezary syndrome, skin cancer, small cell lung cancer, small intestine cancer, supratentorial primitive neuroectodermal tumors, testicular cancer, thymic carcinoma, thymoma, thyroid cancer, transitional cell cancer of the renal pelvis and ureter, trophoblastic tumor, urethral cancer, uterine cancer, vaginal cancer, vulvar cancer, Waldenstrom's macroglobulinemia, and Wilms' tumor.

The disclosed subject matter also concerns methods for treating an infection and/or preventing sepsis in a patient in need thereof. Sepsis is caused by the immune system's response to a serious infection, most commonly bacteria, but also fungi, viruses, and parasites in the blood, urinary tract, lungs, skin, or other tissues.

The disclosed subject matter also concerns methods for treating a subject having an inflammatory and/or autoimmune disorder or condition. MDSC suppress immunity by perturbing both innate and adaptive immune responses. For example, MDSC indirectly affect T cell activation by suppressing CD4⁺ and CD8⁺ T cells by their uptake of arginine and high intracellular level of arginase that depletes their surroundings of arginine, an essential amino acid for T cell activation. In addition, MDSC-produced ROS and peroxynitrite inhibit CD8⁺ T cells by catalyzing the nitration of the TCR and thereby preventing T cell-peptide-MHC interactions. MDSC also perturb tumor immunity by skewing it toward a tumor-promoting type 2 phenotype. They do this by producing the type 2 cytokine IL-10 and by down-regulating macrophage production of the type 1 cytokine IL-12. This effect is amplified by macrophages that increase the MDSC production of IL-10. MDSC accumulation and activation are also identified with chronic inflammation. For example, proinflammatory cytokines IL-1β and IL-6 and the bioactive lipid PGE2 are known to induce MDSC.

Inflammatory and autoimmune disorders or conditions that can be treated by the compounds disclosed include, but are not limited to, systemic lupus erythematosus, Hashimoto's disease, rheumatoid arthritis, gouty arthritis, graft-versus-host disease, Sjögren's syndrome, pernicious anemia, Addison disease, scleroderma, Goodpasture's syndrome, inflammatory bowel diseases such as Crohn's disease, colitis, atypical colitis, chemical colitis; collagenous colitis, distal colitis, diversion colitis: fulminant colitis, indeterminate colitis, infectious colitis, ischemic colitis, lymphocytic colitis, microscopic colitis, gastroenteritis, Hirschsprung's disease, inflammatory digestive diseases, Morbus Crohn, non-chronic or chronic digestive diseases, non-chronic or chronic inflammatory digestive diseases; regional enteritis and ulcerative colitis, autoimmune hemolytic anemia, sterility, myasthenia gravis, multiple sclerosis, Basedow's disease, thrombopenia purpura, insulin-dependent diabetes mellitus, allergy; asthma, atopic disease; arteriosclerosis; myocarditis; cardiomyopathy; glomerular nephritis; hypoplastic anemia; rejection after organ transplantation and numerous malignancies of lung, prostate, liver, ovary, colon, cervix, lymphatic and breast tissues, psoriasis, acne vulgaris, asthma, autoimmune diseases, celiac disease, chronic prostatits, glomerulonephritis, inflammatory bowel diseases, pelvic inflammatory disease, reperfusion injury sarcoidosis, vasculitis, interstitial cystitis, type 1 hypersensitivities, systemic sclerosis, dermatomyositis, polymyositis, and inclusion body myositis.

In one embodiment, an effective amount of one or more compounds or compositions disclosed herein is administered to a subject having an inflammatory or autoimmune disorder and who is in need of treatment thereof. The disclosed methods can optionally include identifying a subject who is or can be in need of treatment of an inflammatory or autoimmune disorder. The subject can be a human or other mammal, such as a primate (monkey, chimpanzee, ape, *etc*.), dog, cat, cow, pig, horse, mouse or other animals having an inflammatory disorder. Means for administering and formulating compounds for administration to a subject are known in the art, examples of which are described herein.

Also disclosed is a method for treating a subject having a neurodegenerative disease or disorder. As used herein, "neurodegenerative disease" includes neurodegenerative disease associated with protein aggregation, also referred to as "protein aggregation disorders", "protein conformation disorders", or "proteinopathies". Neurodegenerative disease associated with protein aggregation include diseases or disorders characterized by the formation of detrimental intracellular protein aggregates (e.g., inclusions in the cytosol or nucleus) or extracellular protein aggregates (e.g., plaques). "Detrimental protein aggregation" is the undesirable and harmful accumulation, oligomerization, fibrillization or aggregation, of two or more, hetero- or homomeric, proteins or peptides. A detrimental protein aggregate may be deposited in bodies, inclusions or plaques, the characteristics of which are often indicative of disease and contain disease- specific proteins. For example, superoxide dismutase-1 aggregates are associated with ALS, poly-Q aggregates are associated with Huntington's disease, and α-synuclein-containing Lewy bodies are associated with Parkinson's disease.

Neurological diseases are also associated with immune failure related to increasing levels of disease-causing factors that exceed the ability of the immune system to contain, or a situation in which immune function deteriorates or is suppressed concomitantly with disease progression, due to factors indirectly or directly related to the disease-causing entity. MDSCs can cause T-cell deficiency by suppressing effector T cell activity, thus promoting neurodegenerative disease associated with immune failure.

Representative examples of Protein Aggregation Disorders or Proteopathies include Protein Conformational Disorders, Alpha-Synucleinopathies, Polyglutamine Diseases, Serpinopathies, Tauopathies or other related disorders. Other examples of neurological diseases or include, but are not limited to, Amyotrophic Lateral Sclerosis (ALS), Huntington's Disease (HD), Parkinson's Disease (PD), Spinal Muscular Atrophy (SMA), Alzheimer's Disease (AD), diffuse Lewy body dementia (DLBD), multiple system atrophy (MSA), dystrophia myotonica, dentatorubro-pallidoluysian atrophy (DRPLA), Friedreich's ataxia, fragile X syndrome, fragile XE mental retardation, Machado-Joseph Disease (MJD or SCA3), spinobulbar muscular atrophy (also known as Kennedy's Disease), spinocerebellar ataxia type 1 (SCA1) gene, spinocerebellar ataxia type 2 (SCA2), spinocerebellar ataxia type 6 (SCA6), spinocerebellar ataxia type 7 (SCA7), spinocerebellar ataxia type 17 (SCA17), chronic liver diseases, familial encephalopathy with neuroserpin inclusion bodies (FENIB), Pick's disease, corticobasal degeneration (CBD), progressive supranuclear palsy (PSP), amyotrophic lateral sclerosis/parkinsonism dementia complex, Cataract, serpinopathies, haemolytic anemia, cystic fibrosis, Wilson's Disease, neurofibromatosis type 2, demyelinating peripheral neuropathies, retinitis pigmentosa, Marfan syndrome, emphysema, idiopathic pulmonary fibrosis, Argyophilic grain dementia, corticobasal degeneration, diffuse neurofibrillary tangles with calcification, frontotemporal dementia/parkinsonism linked to chromosome 17, Hallervorden-Spatz disease, Nieman-Pick disease type C, subacute sclerosing panencephalitis, cognitive disorders including dementia (associated with Alzheimer's disease, ischemia, trauma, vascular problems or stroke, HIV disease, Parkinson's disease, Huntington's disease, Pick's disease, Creutzfeldt-Jacob disease, perinatal hypoxia, other general medical conditions or substance abuse); delirium, amnestic disorders or age related cognitive decline; anxiety disorders including acute stress disorder, agoraphobia, generalized anxiety disorder, obsessive-compulsive disorder, panic attack, panic disorder, post-traumatic stress disorder, separation anxiety disorder, social phobia, specific phobia, substance-induced anxiety disorder and anxiety due to a general medical condition; schizophrenia or psychosis including schizophrenia (paranoid, disorganized, catatonic or undifferentiated), schizophreniform disorder, schizoaffective disorder, delusional disorder, brief psychotic disorder, shared psychotic disorder, psychotic disorder due to a general medical condition and substance-induced psychotic disorder; substance-related disorders and addictive behaviors (including substance-induced delirium, persisting dementia, persisting amnestic disorder, psychotic disorder or anxiety disorder; tolerance, dependence or withdrawal from substances including alcohol, amphetamines, cannabis, cocaine, hallucinogens, inhalants, nicotine, opioids, phencyclidine, sedatives, hypnotics or anxiolytics); movement disorders, including akinesias and akinetic-rigid syndromes (including Parkinson's disease, drug-induced parkinsonism, postencephalitic parkinsonism, progressive supranuclear palsy, corticobasal degeneration, parkinsonism-ALS dementia complex and basal ganglia calcification), medication-induced parkinsonism (such as neuroleptic-induced parkinsonism, neuroleptic malignant syndrome, neuroleptic-induced acute dystonia, neuroleptic-induced acute akathisia, neuroleptic-induced tardive dyskinesia and medication-induced postural tremor), Gilles de la Tourette's syndrome, epilepsy, and dyskinesias including tremor (such as rest tremor, postural tremor and intention tremor), chorea (such as Sydenham's chorea, Huntington's disease, benign hereditary chorea, neuroacanthocytosis, symptomatic chorea, drug-induced chorea and hemiballism), myoclonus (including generalized myoclonus and focal myoclonus), tics (including simple tics, complex tics and symptomatic tics), and dystonia (including generalized dystonia such as iodiopathic dystonia, drug-induced dystonia, symptomatic dystonia and paroxysmal dystonia, and focal dystonia such as blepharospasm, oromandibular dystonia, spasmodic dysphonia, spasmodic torticollis, axial dystonia, dystonic writer's cramp and hemiplegic dystonia)]; obesity, bulimia nervosa and compulsive eating disorders; pain including bone and joint pain (osteoarthritis), repetitive motion pain, dental pain, cancer pain, myofacial pain (muscular injury, fibromyalgia), perioperative pain (general surgery, gynecological), chronic pain, neuropathic pain, post-traumatic pain, trigeminal neuralgia, migraine and migraine headache; obesity or eating disorders associated with excessive food intake and complications associated therewith; attention-deficit/hyperactivity disorder; conduct disorder; mood disorders including depressive disorders, bipolar disorders, mood disorders due to a general medical condition, and substance-induced mood disorders; muscular spasms and disorders associated with muscular spasticity or weakness including tremors; urinary incontinence; amyotrophic lateral sclerosis; neuronal damage including ocular damage, retinopathy or macular degeneration of the eye, hearing loss or tinnitus; emesis, brain edema and sleep disorders including narcolepsy, and apoptosis of motor neuron cells. Illustrative examples of the neuropathic pain include diabetic polyneuropathy, entrapment neuropathy, phantom pain, thalamic pain after stroke, post-herpetic neuralgia, atypical facial neuralgia pain after tooth extraction and the like, spinal cord injury, trigeminal neuralgia and cancer pain resistant to narcotic analgesics such as morphine. The neuropathic pain includes the pain caused by either central or peripheral nerve damage. And it includes the pain caused by either mononeuropathy or polyneuropathy.

Further provided herein are methods of treating anemia of chronic disease (including cancer-related anemia) in a subject, comprising administering to the subject an effective amount of a compound or composition as disclosed herein.

### Compositions, Formulations and Methods of Administration

*In vivo* application of the disclosed compounds, and compositions containing them, can be accomplished by any suitable method and technique presently or prospectively known to those skilled in the art. For example, the disclosed compounds can be formulated in a physiologically- or pharmaceutically-acceptable form and administered by any suitable route known in the art including, for example, oral, nasal, rectal, topical, and parenteral routes of administration. As used herein, the term parenteral includes subcutaneous, intradermal, intravenous, intramuscular, intraperitoneal, and intrasternal administration, such as by injection. Administration of the disclosed compounds or compositions can be a single administration, or at continuous or distinct intervals as can be readily determined by a person skilled in the art.

The compounds disclosed herein, and compositions comprising them, can also be administered utilizing liposome technology, slow release capsules, implantable pumps, and biodegradable containers. These delivery methods can, advantageously, provide a uniform dosage over an extended period of time. The compounds can also be administered in their salt derivative forms or crystalline forms.

The compounds disclosed herein can be formulated according to known methods for preparing pharmaceutically acceptable compositions. Formulations are described in detail in a number of sources which are well known and readily available to those skilled in the art. For example, Remington's Pharmaceutical Science by E.W. Martin (1995) describes formulations that can be used in connection with the disclosed methods. In general, the compounds disclosed herein can be formulated such that an effective amount of the compound is combined with a suitable carrier in order to facilitate effective administration of the compound. The compositions used can also be in a variety of forms. These include, for example, solid, semi-solid, and liquid dosage forms, such as tablets, pills, powders, liquid solutions or suspension, suppositories, injectable and infusible solutions, and sprays. The preferred form depends on the intended mode of administration and therapeutic application. The compositions also preferably include conventional pharmaceutically-acceptable carriers and diluents which are known to those skilled in the art. Examples of carriers or diluents for use with the compounds include ethanol, dimethyl sulfoxide, glycerol, alumina, starch, saline, and equivalent carriers and diluents. To provide for the administration of such dosages for the desired therapeutic treatment, compositions disclosed herein can advantageously comprise between about 0.1% and 100% by weight of the total of one or more of the subject compounds based on the weight of the total composition including carrier or diluent.

Formulations suitable for administration include, for example, aqueous sterile injection solutions, which can contain antioxidants, buffers, bacteriostats, and solutes that render the formulation isotonic with the blood of the intended recipient; and aqueous and nonaqueous sterile suspensions, which can include suspending agents and thickening agents. The formulations can be presented in unit-dose or multi-dose containers, for example sealed ampoules and vials, and can be stored in a freeze dried (lyophilized) condition requiring only the condition of the sterile liquid carrier, for example, water for injections, prior to use. Extemporaneous injection solutions and suspensions can be prepared from sterile powder, granules, tablets, *etc.* It should be understood that in addition to the ingredients particularly mentioned above, the compositions disclosed herein can include other agents conventional in the art having regard to the type of formulation in question.

Compounds disclosed herein, and compositions comprising them, can be delivered to a cell either through direct contact with the cell or via a carrier means. Carrier means for delivering compounds and compositions to cells are known in the art and include, for example, encapsulating the composition in a liposome moiety. Another means for delivery of compounds and compositions disclosed herein to a cell comprises attaching the compounds to a protein or nucleic acid that is targeted for delivery to the target cell. U.S. Patent No. 6,960,648 and U.S. Application Publication Nos. 20030032594 and 20020120100 disclose amino acid sequences that can be coupled to another composition and that allows the composition to be translocated across biological membranes. U.S. Application Publication No. 20020035243 also describes compositions for transporting biological moieties across cell membranes for intracellular delivery. Compounds can also be incorporated into polymers, examples of which include poly (D-L lactide-co-glycolide) polymer for intracranial tumors; poly[bis(p-carboxyphenoxy) propane :sebacic acid] in a 20:80 molar ratio (as used in GLIADEL); chondroitin; chitin; and chitosan.

For the treatment of oncological disorders, the compounds disclosed herein can be administered to a patient in need of treatment in combination with other antitumor or anticancer substances and/or with radiation and/or photodynamic therapy and/or with surgical treatment to remove a tumor. These other substances or treatments can be given at the same as or at different times from the compounds disclosed herein. For example, the compounds disclosed herein can be used in combination with mitotic inhibitors such as taxol or vinblastine, alkylating agents such as cyclophosamide or ifosfamide, antimetabolites such as 5-fluorouracil or hydroxyurea, DNA intercalators such as adriamycin or bleomycin, topoisomerase inhibitors such as etoposide or camptothecin, antiangiogenic agents such as angiostatin, antiestrogens such as tamoxifen, and/or other anti-cancer drugs or antibodies, such as, for example, GLEEVEC (Novartis Pharmaceuticals Corporation) and HERCEPTIN (Genentech, Inc.), respectively, or an immunotherapeutic such as ipilimumab and bortezomib. In other aspect, the disclosed compounds are coadministered with other HDAC inhibitors like ACY-1215, Tubacin, Tubastatin A, ST-3-06, OR ST-2-92.

In certain examples, compounds and compositions disclosed herein can be locally administered at one or more anatomical sites, such as sites of unwanted cell growth (such as a tumor site or benign skin growth, *e.g.,* injected or topically applied to the tumor or skin growth), optionally in combination with a pharmaceutically acceptable carrier such as an inert diluent. Compounds and compositions disclosed herein can be systemically administered, such as intravenously or orally, optionally in combination with a pharmaceutically acceptable carrier such as an inert diluent, or an assimilable edible carrier for oral delivery. They can be enclosed in hard or soft shell gelatin capsules, can be compressed into tablets, or can be incorporated directly with the food of the patient's diet. For oral therapeutic administration, the active compound can be combined with one or more excipients and used in the form of ingestible tablets, buccal tablets, troches, capsules, elixirs, suspensions, syrups, wafers, aerosol sprays, and the like.

The tablets, troches, pills, capsules, and the like can also contain the following: binders such as gum tragacanth, acacia, corn starch or gelatin; excipients such as dicalcium phosphate; a disintegrating agent such as corn starch, potato starch, alginic acid and the like; a lubricant such as magnesium stearate; and a sweetening agent such as sucrose, fructose, lactose or aspartame or a flavoring agent such as peppermint, oil of wintergreen, or cherry flavoring can be added. When the unit dosage form is a capsule, it can contain, in addition to materials of the above type, a liquid carrier, such as a vegetable oil or a polyethylene glycol. Various other materials can be present as coatings or to otherwise modify the physical form of the solid unit dosage form. For instance, tablets, pills, or capsules can be coated with gelatin, wax, shellac, or sugar and the like. A syrup or elixir can contain the active compound, sucrose or fructose as a sweetening agent, methyl and propylparabens as preservatives, a dye and flavoring such as cherry or orange flavor. Of course, any material used in preparing any unit dosage form should be pharmaceutically acceptable and substantially non-toxic in the amounts employed. In addition, the active compound can be incorporated into sustained-release preparations and devices.

Compounds and compositions disclosed herein, including pharmaceutically acceptable salts or prodrugs thereof, can be administered intravenously, intramuscularly, or intraperitoneally by infusion or injection. Solutions of the active agent or its salts can be prepared in water, optionally mixed with a nontoxic surfactant. Dispersions can also be prepared in glycerol, liquid polyethylene glycols, triacetin, and mixtures thereof and in oils. Under ordinary conditions of storage and use, these preparations can contain a preservative to prevent the growth of microorganisms.

The pharmaceutical dosage forms suitable for injection or infusion can include sterile aqueous solutions or dispersions or sterile powders comprising the active ingredient, which are adapted for the extemporaneous preparation of sterile injectable or infusible solutions or dispersions, optionally encapsulated in liposomes. The ultimate dosage form should be sterile, fluid and stable under the conditions of manufacture and storage. The liquid carrier or vehicle can be a solvent or liquid dispersion medium comprising, for example, water, ethanol, a polyol (for example, glycerol, propylene glycol, liquid polyethylene glycols, and the like), vegetable oils, nontoxic glyceryl esters, and suitable mixtures thereof. The proper fluidity can be maintained, for example, by the formation of liposomes, by the maintenance of the required particle size in the case of dispersions or by the use of surfactants. Optionally, the prevention of the action of microorganisms can be brought about by various other antibacterial and antifungal agents, for example, parabens, chlorobutanol, phenol, sorbic acid, thimerosal, and the like. In many cases, it will be preferable to include isotonic agents, for example, sugars, buffers or sodium chloride. Prolonged absorption of the injectable compositions can be brought about by the inclusion of agents that delay absorption, for example, aluminum monostearate and gelatin.

Sterile injectable solutions are prepared by incorporating a compound and/or agent disclosed herein in the required amount in the appropriate solvent with various other ingredients enumerated above, as required, followed by filter sterilization. In the case of sterile powders for the preparation of sterile injectable solutions, the preferred methods of preparation are vacuum drying and the freeze drying techniques, which yield a powder of the active ingredient plus any additional desired ingredient present in the previously sterile-filtered solutions.

For topical administration, compounds and agents disclosed herein can be applied in as a liquid or solid. However, it will generally be desirable to administer them topically to the skin as compositions, in combination with a dermatologically acceptable carrier, which can be a solid or a liquid. Compounds and agents and compositions disclosed herein can be applied topically to a subject's skin to reduce the size (and can include complete removal) of malignant or benign growths, or to treat an infection site. Compounds and agents disclosed herein can be applied directly to the growth or infection site. Preferably, the compounds and agents are applied to the growth or infection site in a formulation such as an ointment, cream, lotion, solution, tincture, or the like.

Useful solid carriers include finely divided solids such as talc, clay, microcrystalline cellulose, silica, alumina and the like. Useful liquid carriers include water, alcohols or glycols or water-alcohol/glycol blends, in which the compounds can be dissolved or dispersed at effective levels, optionally with the aid of non-toxic surfactants. Adjuvants such as fragrances and additional antimicrobial agents can be added to optimize the properties for a given use. The resultant liquid compositions can be applied from absorbent pads, used to impregnate bandages and other dressings, or sprayed onto the affected area using pump-type or aerosol sprayers, for example.

Thickeners such as synthetic polymers, fatty acids, fatty acid salts and esters, fatty alcohols, modified celluloses or modified mineral materials can also be employed with liquid carriers to form spreadable pastes, gels, ointments, soaps, and the like, for application directly to the skin of the user.

Useful dosages of the compounds and agents and pharmaceutical compositions disclosed herein can be determined by comparing their *in vitro* activity, and *in vivo* activity in animal models. Methods for the extrapolation of effective dosages in mice, and other animals, to humans are known to the art.

Also disclosed are pharmaceutical compositions that comprise a compound disclosed herein in combination with a pharmaceutically acceptable carrier. Pharmaceutical compositions adapted for oral, topical or parenteral administration, comprising an amount of a compound constitute a preferred aspect. The dose administered to a patient, particularly a human, should be sufficient to achieve a therapeutic response in the patient over a reasonable time frame, without lethal toxicity, and preferably causing no more than an acceptable level of side effects or morbidity. One skilled in the art will recognize that dosage will depend upon a variety of factors including the condition (health) of the subject, the body weight of the subject, kind of concurrent treatment, if any, frequency of treatment, therapeutic ratio, as well as the severity and stage of the pathological condition.

Also disclosed are kits that comprise a composition comprising a compound disclosed herein in one or more containers. The disclosed kits can optionally include pharmaceutically acceptable carriers and/or diluents. In one embodiment, a kit includes one or more other components, adjuncts, or adjuvants as described herein. In another embodiment, a kit includes one or more anti-cancer agents, such as those agents described herein. In one embodiment, a kit includes instructions or packaging materials that describe how to administer a compound or composition of the kit. Containers of the kit can be of any suitable material, *e.g.,* glass, plastic, metal, *etc.,* and of any suitable size, shape, or configuration. In one embodiment, a compound and/or agent disclosed herein is provided in the kit as a solid, such as a tablet, pill, or powder form. In another embodiment, a compound and/or agent disclosed herein is provided in the kit as a liquid or solution. In one embodiment, the kit comprises an ampoule or syringe containing a compound and/or agent disclosed herein in liquid or solution form.

### EXAMPLES

The following examples are set forth below to illustrate the methods and results according to the disclosed subject matter. These examples are not intended to be inclusive of all aspects of the subject matter disclosed herein, but rather to illustrate representative methods and results. These examples are not intended to exclude equivalents and variations of the present invention which are apparent to one skilled in the art.

Efforts have been made to ensure accuracy with respect to numbers (*e.g.,* amounts, temperature, etc.) but some errors and deviations should be accounted for. Unless indicated otherwise, parts are parts by weight, temperature is in °C or is at ambient temperature, and pressure is at or near atmospheric. There are numerous variations and combinations of reaction conditions, *e.g.,* component concentrations, temperatures, pressures and other reaction ranges and conditions that can be used to optimize the product purity and yield obtained from the described process. Only reasonable and routine experimentation will be required to optimize such process conditions.

### Example 1: ICA/ICT prevent tumor growth in vivo by modulating MDSC

One of the most striking features of ICA and ICT is their anti-tumorigenic ability *in vivo.* Seven days post-infection 1 cm diameter 4T1 tumors from 5 mice were established in BALB/c mice. Mice were treated with 100 mg/kg of ICA, ICT or vehicle (i.p.) three times weekly and tumor size (mean and SD) was monitored every 2 to 3 days. The percentages of double-positive Gr-1⁺CD11b⁺ MDSCs were determined for spleens of five mice per group. Cell phenotype was evaluated by flow cytometry. It is found that ICA and ICT can significantly down-regulate the percent of circulating MDSC from 50.28% to 33.35% and 26.97%, respectively (**Fig. 1**). It is found that ICA and ICT can inhibit tumor growth *in vivo* by decreasing the accumulation and activation of circulating MDSC (Zhou J et al. Int Immunopharmacol. 2011;11(7):890-8; Wu J et al. Int Immunopharmacol. 2011;12(1):74-9). ICA or ICT on the differentiation of MDSC.

Furthermore, the toxicity of these compounds was tested on tumor cells and immune cells and it was found that ICA and ICT are not toxic to cells of hematopoietic or myeloid origin such as PBMCs, bone marrow mononuclear cells (BMMC), or U937 cells (IC₅₀ > 100 µM) and do not affect hematopoiesis (**Fig. 2**). Specifically, BMNCs from three healthy donors were treated with different concentrations of ICA or ICT for 48 hours after which colony formation was assessed using MethoCult H4434 complete medium with cytokines. The mixture was placed in 35-mm culture dishes (1 × 10⁵ cells/each dish) and incubated at 37°C in 5% CO₂ for approximately 7-14 days. After incubation, colonies of CFU-E, BFU-E and CFU-GM were indentified and counted using an inverted light microscope. This observation demonstrates a strong correlation between the down regulation of MDSCs in the tumor microenvironment and treatment with ICA, ICT, and the derivatives disclosed herein. Although, these cells were obtained from spleens, it was also found ICA/ICT has a similar effect on MDSCs isolated from tumor tissue. Here, Gr1⁺ cells were isolated from tumors of 4T1 tumor-bearing mice. MDSCs were treated with 20 µM of ICA, ICT, or DMSO for 48h. Bars represent the relative percentage of MDSC as compared to DMSO treated cells measured by flow cytometry (**Fig. 3A**). The production of ROS (**Fig. 3B**) and NO (**Fig. 3C****)** activity of purified MDSC from tumor tissue was also measured.

### Example 2: ICA and ICT can inhibit SIGLEC3/CD33 expression in human PBMCs

SIGLEC3 is highly expressed in MDSCs isolated from patients with AML (**Fig. 6a**). It was found that ICA and ICT are capable of down-regulating SIGLEC3 at both the mRNA and protein expression levels (**Fig. 4** and **5**) (Zhou J et al. Int Immunopharmacol. 2011;11(7):890-8). Specifically, healthy PBMC were cultured for 48 hours with 1 µM of DMSO, ICA or ICT and the expression of SIGLEC3 was measured by Q-PCR and analyzed by the ΔΔCt method. Also, PBMC from healthy donors were treated for 48 hours with DMSO or ICT and stained extra-cellularly with SIGLEC3-PE and SIGLEC5/14-APC antibodies (BD biosciences). S100A9 can ligate with SIGLEC3, denoting the presence of an uncharacterized pathway that could be linked to the identification of specific strategies targeting the regulation of MDSC activation.

### Example 3: Identification of S100A9 as an endogenous ligand for SIGLEC3

PDE5, and its interaction with the S100A9 pathway, can be essential for the accumulation and activation of MDSC in tumors, but their specific upstream pathways are poorly understood. It has been demonstrated that there is a remarkable increase in SIGLEC3 in the MDSCs of patients with leukemia (**Fig. 10a**) (Wei S, et al. ASH Annual Meeting Abstracts. 2009;114(22):597). SIGLEC3 is a marker for MDSCs and can mediate suppressive signaling through its ITIM motifs, although its relevant ligand or the pathways below it remain unknown. Therefore, chimeras of the ectodomain of SIGLEC3 were created with human IgG-Fc and the chimeras were used it to immune-precipitate ligands from the lysate of MDSCs isolated from the bone marrow (BM) of patients with MDS (Myelodysplastic syndrome, a premalignant disorder that transforms to AML (acute myeloid leukemia)) and analyzed by mass spectrometry. The most prominent band was between 10 and 15 kDa and, moreover, one of the prominent hits after mass spectrometry was S100A9. Since a correlation between SINGLE3 and S1-A9 has been established, an *in vitro* direct binding assay was performed to corroborate the affinity between these two components in an isolated system. As seen in **Fig. 7****,** the SIGLEC3 chimera directly bound S100A9 expressed on transfected AD293 cells (similar results were obtained on SJCRH30 rhabdomysosarcoma cells, a cell line lacking expression of SIGLEC3. Furthermore, immune-precipitating S100A9 showed direct specificity for SIGLEC3 and moreover cells that express this receptor, such as the MDSCs from MDS patients, can bind recombinant human (rh) S100A9 as demonstrated by its co-localization with SIGLEC3 (**Fig. 8** and **Fig. 6b**). Interestingly, S100A8, which normally pairs with S100A9, came out only as part of a hetero-multimer, but not individually, indicating that S100A9 can be the actual ligand for SIGLEC3.

### Example 4: S100A9/SIGLEC3 can be linked to both PDE5 and Protein phosphatase 2A (PP2A) activity in MDSC and can be regulated by ICA and ICT

ICA/ICT can up-regulate the phosphatase activity of PP2A *in vitro* (**Fig. 9**) which correlates with the de-phosphorylation of NF-κB and MAPK after treatment (Zhou J et al. Int Immunopharmacol. 2011;11(7):890-8). A direct association or binding of these two proteins was tested for, as it has recently been demonstrated for PP1 (Murthy KS. Br J Pharmacol. 2008;153(6):1214-24). Unexpectedly, it was found that PP2A is constitutively bound to PDE5 as demonstrated by co-immuno-precipitation of PDE5 following Western blotting with PP2A (**Fig. 10**). To understand if there is a link correlating S100A9/SIGLEC3 and PDE5 activation, SJCRH30 cells were transfected with either vector, S100A9 or SIGLEC 3. Seventy two hours post-transfection cells were lysed and assessed by Western Blot for either PDE5 or its phosphorylated counterpart. Overexpression of SIGLEC3 up-regulated the activation of PDE5 (increased PDE5 activation, demonstrated by its enhanced phosphorylation on Ser92 in PDE5, which can be detected by Western Blot using antibody specific to phospho-Ser92) although its expression remained unchanged (**Fig. 11**). This represents a new pathway of MDSC activation that can be specifically targeted by ICA, ICT, and their derivatives disclosed herein.

### Example 5: Modeling the binding mode of ICA and ICT to PDE5

ICA and ICT can have similar effects to other PDE5 inhibitors, but it has not been demonstrated if they are capable of binding to the inhibitory location in the PDE5 molecule. A metabolite of ICA, icarisid II, has a co-crystal structure and model building was used to demonstrate the binding affinities of ICA and ICT compounds for this site.

The crystal structure used herein was 2H44, obtained from the Protein Data Bank. It features PDE5A1 complexed with Icarisid II at 1.0Å resolution (Wang H et al. J. Biol. Chem. 2006;281(30):21469-79). Small molecule structures were obtained from PubChem and include: icariin (CID: 5318997), icaritin (CID: 5318980), and icarisid II (CID: 6852214); ICT (3,5,7-Trihydroxy- 4'-methoxy-8- (3-hydroxy- 3 methylbutyl)- flavone) was generated from the icaritin structure.

Schrodinger's Discovery Suite (Schrodinger, L.L.C.) was used for docking studies. LigPrep 2.4 (Schrodinger, L.L.C.) was used for preparation of the structure library including generation of tautomers and alternative ionization states using Epik for pH values ranging from 5.0 to 9.0 and for generation of stereoisomers as needed. Schrodinger's GLIDE 5.6 (Schrodinger, L.L.C.) was used for generating receptor grids for 2H44 and for docking using standard precision (SP) followed by extra precision (XP). Sitemap 2.4 (Schrodinger, L.L.C.) was used to determine the chemical nature of the binding site of 2H44 including: hydrophobic regions, hydrogen bond donating regions, and hydrogen bond accepting regions. 2H44 was prepared for modeling studies using the Protein Prep workflow which corrects side chains due to missing atoms, optimizes hydrogen bonds within the protein, removes water molecules, and performs a constrained energy minimization to an RMSD of no greater than 0.30Å using the OPLS-2005 force field.

The 2H44 crystal structures were prepared as described above with default values. Receptor grids were generated using the co-crystallized ligand, Icarisid II, to determine the grid center. Other settings were left at default values with the exception of grid size, which was set to maximum. Icarisid II was extracted from the 2H44 crystal structure and was prepared by using LigPrep. A total of 32 structures were obtained and formed the test library for docking simulation to verify the capability of the model to reproduce the co-crystallized pose of Icarisid II in 2H44 (**Fig. 12A**). GLIDE was used with default settings to run a standard precision (SP) docking followed by an extra-precision refinement (XP) of Icarisid II to PDE5A1. The lowest energy pose has a GScore of -17.22 kcal/mol and superimposes with the co-crystal position of Icarisid II (RMS of 0.9558, **Fig. 12A**). The hydrogen bonds are identified at S668, H613, and 1665, all of which are present in the original crystallographic pose. These results provide confidence that the model is suitable for predicting binding poses of molecules related to Icarisid II.

ICT was generated by modifying a previous structure (5318980 PubChem structure). ICT was prepared for docking using LigPrep as described with Icarisid II. As with the control docking an SP docking with an XP refinement was executed, using the ICT LigPrep results (one structure only) as the ligand library and the receptor grid previously generated for PDE5A1 in the Icarasid II docking. A single pose was obtained from the XP dock, which has a glide Gscore of -13.013 Kcal/mol. This pose presents three hydrogen bond interactions S663, H613, D764 (dotted lines) and potential pi stacking with F820 (parallel dotted lines) (**Fig. 12B**). This result provides the information needed to perform optimization of the ligands. To support the validity of the predicted *in silico* interaction, it was demonstrated *in vitro* that both ICA and ICT can block the phosphodiesterase activity of PDE5 with a cyclic nucleotide phosphodiesterase assay kit (Promega Corp).

### Example 6: Activation of inflammatory MDSCs via S100A9 binding to SIGLEC3/CD33 receptor

The downstream signaling events following the ligation of SIGLEC3 with S100A9 can be assessed using IMC (CD33⁺HLA-DR⁻Lin⁻) isolated from the bone marrow of healthy donors (purchased from Lonza Walkersville Inc. Walkersville, MD). Recombinant human S100A9-DDK can be used to stimulate either PBMC or IMC for 15, 30, 45 or 60 minutes after which they can be cytospinned and stained with a fluorescently conjugated anti-MYC and anti-SIGLEC3 antibodies. Co-localization of the two proteins and the internal location of the complex after binding can then be observed. SIGLEC3 can be prepared in adenoviral or lentiviral (LV) vectors and can be overexpressed in either IMC or SJCRH30 cells (as done in **Fig. 7** and **Fig. 8**) before treatment with rhS100A9-DDK for 24, 48, or 72 hours. This strategy can be used to confirm if signaling through S100A9/SIGLEC3 can activate IMC and induce their differentiation into MDSC. After engagement of S100A9 with SIGLEC3, culture supernatants can be collected and analyzed for production of the suppressive soluble factors: TGFβ, IL-10, VEGF, ROS and NO. The expression of SIGLEC3, PDE5, PKC and PP2A can be tested by QPCR, and their activation can be measured by either phosphorylation status or activation by Western Blot analysis. In order to assess if S100A9's calcium binding activity has an effect on its binding affinity to the receptor and to understand the role of sugar ligation, a chelating agent such as EDTA or a glycosidase (to remove the sugar moieties of S100A9) can be added. Upon ligation with S100A9, IMC can be activated and behave as MDSC and provide clear evidence that S1009/SIGLEC3 ligation can be crucial for MDSC activation. Following a similar strategy, IMCs that express SIGLEC3 can be treated with ICA/ICT before ligation with S100A9 to examine if this pathway can be modulated by ICA/ICT. Upon treatment with these compounds, S100A9/SIGLEC3 mediated signaling can be blocked, which will lead to a reduced IMC-MDSC transition, decreased suppressive cytokine production and NO/ROS production as compared to DMSO treated cells.

To assess the responsibility of the intracellular ITIM domain in SIGLEC3 signaling of MDSC, several CD33 dominant-negative mutants of the ITIM tyrosine site (CD33^{Y340F}, CD33^{Y358F} and CD33^{Y340/Y358F}) can be created (tyrosine substitution by phenylalanine or by alanine). These mutants and wild type (wt) SIGLEC3 can be transfected into AD293 or SJCRH30 cells. These rhabdomyosarcoma cells do not express endogenous SIGLEC3 and S100A9. Three major downstream signaling functions can be assessed in these SIGLEC3 expressing cells after ligation with rhS100A9. First, the MAPK/ERK activation can be assessed using Western Blot analysis with anti-phospho-MAPK. MAPKs can be a downstream target of ITIM signaling (Yoder JA et al. Proc Natl Acad Sci USA. 2001;98(12):6771-6). Specificity of SIGLEC3 signaling can be controlled using an isotype antibody or non-relevant ligand such as S100A7. Controls can also include AD293 or SJCRH30 cells cultured with media alone, transfected with control vector and ligation with non-relevant ligands without transfection of SIGLEC. S100A9/SIGLEC3 signaling in wt-SIGLEC3 transfected cells can recruit SHP1/2 reducing the phosphorylation level of MAPK. However, in the SIGLEC3 mutant groups cross-linking SIGLEC3 can fail to recruit the SHP1/2 phosphatases and result in either increased or persistent levels of phosphorylation of MAPK. Second, ITIM signaling in SIGLEC3 transfectants can be directly measured following ligation with S100A9. MDSC employ SIGLEC-associated ITIMs to mediate their suppressive function. After engagement of SIGLEC3 with S100A9, the tyrosines in the ITIMs can become phosphorylated and recruit and activate tyrosine phosphatases SHP1/SHP2. Therefore, the phosphorylation of ITIM motifs or recruitment of cytoplasmic tyrosine phosphatases can be measured by immune-precipitation of SIGLEC3 using anti-CD33 mAb, followed by Western Blot analysis with anti-phospho-tyrosine or anti-phosphatase antibodies respectively. Third, SIGLEC3 transfectants can be cultured for 48-72 hours and supernatants can be assessed for TGFβ, IL-10 and VEGF production as well as ROS and NO production. Specific cross-linking of SIGLEC3 can lead to increased TGFβ, IL-10 and VEGF production. Over-expressed wt-SIGLEC3 in these cells can also increase these cytokines. In contrast, SIGLEC3 mutant-transfected cells can display decreased suppressive cytokine production due to the lack of ITIM signaling in these cells.

To confirm the results in primary cells the molecular mechanism of S100A9/SIGLEC3 signaling in human IMCs from healthy donors can be examined. For this, all SIGLEC3 constructs can be converted into lentiviral vectors (LV) to express in IMCs. The LV-SIGLEC3 constructs can be introduced into IMCs and, after cross-linking with S100A9, can be used: 1) to assess cytokines, ROS and NO production, 2) to monitor long term (14 days) over expression of wt-SIGLEC3 in IMCs, which will block maturation of myeloid cell and induce MDSC accumulation.

ITIM phosphorylation can be measured to determine the impact of ICA/ICT on SIGLEC3 signaling in its transfectants. After treatment of cells with ICA/ICT at various concentration and time, the phosphorylation of ITIMs and the recruitment of phosphatases can be measured by immune-precipitation of the SIGLEC3 receptors followed by Western Blot analysis using anti-phosphotyrosine or anti-phosphatase antibodies, respectively. This strategy can reveal whether or not SIGLEC3 signaling is responsible for ICA/ICT function and/or confirm if ICA/ICT can disrupt S100A9/SIGLEC3 signaling pathway at the receptor level.

Because SIGLEC3 is overexpressed on MDSCs from tumor tissues (Fig. 6a), they can be used to test the signaling events mediated by S100A9/SIGLEC3 with or without treatment with ICA/ICT. This can be used to examine the expression level of S100A9 and SIGLEC3 in MDSC isolated from cancer; the suppressive cytokine production (e.g. TGFβ, IL-10 etc.); ROS and NO production in these MDSCs; and changes of downstream molecules of S100A9/SIGLEC3 signaling. Based on the effects of ICA/ICT previously observed, the activation of MAPK, PI3K-AKT, STAT3 and TLR4 in MDSCs can be tested due to their role as components of the SIGLEC3/S100A9 suppressive pathways.

It has been reported that S100A8/A9 can activate TLR4 *in vivo* (Ehrchen JM et al. J Leukoc Biol. 2009;86(3):557-66). Thus, ICA/ICT and their derivatives disclosed herein can inhibit TLR4 signaling and may directly or indirectly affect both the TLR4 and SIGLEC3 pathways. This can help identify the specific pathways targeted by ICA/ICT and gain knowledge of how the SIGLEC3 and TLR4 pathways orchestrate MDSC activation during inflammation. Several approaches can be used to investigate how SIGLEC3 and TLR4 may cross-talk in response to treatment with these agents, including NF-κB and MyD88 deficient mice. An RNAi strategy can be used to address this question. LV vectors containing shRNA (LV-shRNA) can be used to knockdown specific signaling proteins in order to examine the downstream events after ICA/ICT treatment and compare the effect of these compounds on both pathways. Specific shRNA to S100A9, SIGLEC3, TLR4 and MyD88 can be designed and constructed into LV vectors. This LV-shRNA can be used to force-silence of these genes to determine if their expression is directly linked to S100A9/SIGLEC3 or TLR4 signaling. Transfections of viral vector/mock-infected cells, GFP-containing vector or non-targeted shRNA can be used as controls. The transfection rate and protein specific expression can be verified by FACS, Western Blot, and Q-PCR. After protein specific knockdown to either SIGLEC3 or TLR4 pathway using the above mentioned shRNAs, the cells can be treated with either rhS100A9 for SLGLEC signaling or with LPS for TLR4 signaling before measuring the downstream signal event. S100A7 can be used as a nonspecific control. Specific shRNA can disrupt either the S100A9/SIGLEC3 signaling or TLR signaling. At this point, these TLR4 or SIGLEC3 deficient cells can be used to determine if they are able to respond directly or indirectly to ICT/ICA treatment by measuring their downstream MDSC activities. This can provide valuable insight as to the signal transduction pathways responsible not only for MDSC activation but also for ICA/ICT response. The TLR4 activity can be assessed by measuring the levels of phospho-IκBα or NF-κB nuclear translocation.

### Example 7: Role of PDE5 inhibition by PP2A on the deactivation of pro-inflammatory mediators by ICA and ICT

RNS are potent inflammatory mediators in MDSCs and it is known to be under the control of iNOS, an enzyme induced by Phosphodiesterase-5 (PDE5). Thus, activated PDE5 can induce a robust production of NO by MDSCs to mediate immune suppression. PDE5 can be induced by S100A9/SIGLEC3 ligation. PDE5 inhibition in MDSCs, e.g. with Sildenafil, can be effective in preventing tumor growth in mice; an action that can be traced to the suppression of MDSC *in vivo* (Serafini P, et al. J Exp Med. 2006;203(12):2691-702). Therefore, PDE5, which lies upstream of iNOS, can be a factor for MDSC activation and function.

ICA/ICT and their derivatives disclosed herein can have the same effect as Sildenfil in reducing MDSCs and iNOS, and ICA/ICT can be working via PDE5 inhibition to achieve this outcome. Indeed, ICA and ICT can bind to PDE5 (**Fig. 12**). ICA/ICT can inhibit NO and ROS production by MDSCs isolated from the tissue of tumor-bearing mice, as well as the accumulation of MDSC at the tumor site (**Fig. 3**). Therefore, ICA/ICT and their derivatives disclosed herein can act through the modulation of PDE5 activity in MDSCs.

MDSC can be isolated as described above and treated with LV-shRNA specific to PDE5 or PDE4 (a phosphodiesterase not affected by PDE5 inhibitors) to confirm whether knocking down PDE5 alone is sufficient to reduce the functional activation of MDSC. Next, LV-PDE5 can be overexpressed in MDSC, IMC or SJCRH30 followed by ICA/ICT treatment. Overall MDSC biological function can be evaluated to address how ICA/ICT interacts with PDE5. The transfected cells can be treated with ICA/ICT at different doses (1, 5, 10 and 20 µM) for different times (24, 48 or 72 hours). The cells can then be analyzed for: a) the expression of iNOS, TGFβ and IL-10 by ELISA and Q-PCR; b) the phosphorylation status of inflammation markers including IκBα, p38, AKT and STAT3 by Western Blot; c) IMC maturation status (overexpression of PDE5 can block IMC maturation and promote expansion of MDSC); d) T cell suppression by co-culturing treated MDSCs at different ratios with stimulated (with anti-CD3 and CD28 or PHA-activated (1 µg/ml)) autologous CFSE-labeled T cells. Reduced T cell proliferation is one indicator of MDSC activation and ICA/ICT could improve T cell proliferation due to its inhibition of MDSC by blocking PDE5 activity. Therefore, knocking down PDE5 can replicate the effect of ICA/ICT while overexpression of PDE5 can validate the effect of both PDE5 and ICA/ICT on MDSC.

Although SIGLEC3 can be indirectly linked to PDE5, their relationship has not been directly studied. There is evidence that PDE5's response to S100A9/SIGLEC3 signaling can be an increased activation (**Fig. 11**). It has also been shown that PDE5 can be constitutively associated with its regulator, Phosphatase-2A (PP2A), which can de-phosphorylate the serine 92 of PDE5, an association decreased after ICA/ICT treatment (**Fig. 10**). ICA/ICT can significantly up-regulate PP2A enzymatic activity (**Fig. 9**) indicating that, besides direct inhibition of PDE5, they can alternatively stimulate PP2A to suppress PDE5. PP2A, best known as a tumor suppressor and an inhibitor of cell growth, can cause target cells to undergo apoptosis when activated. It is also well established that this phosphatase is downregulated in cancer patients and therefore its expression and activity can be reduced in MDSC and can be a part of their suppressive mechanism. Phosphorylation of tyr-307 on the catalytic subunit of PP2A (PP2A*ca*) can be responsible for the inactivation of its enzymatic activity. Therefore, the phosphorylation status of tyr-307 of PP2A from MDSC can be assessed by Western Blot after ligation with S100A9 to examine if PP2A activity is reduced in MDSC. Subsequently, it can be examined if decreased PP2A activity in MDSC can be rescued by ICA/ICT treatment. Co-immune-precipitation with anti-SIGLEC3 can be performed to confirm if PP2A, PDE5 and SIGLEC3 coexist in the same complex. Phosphorylation of Ser92 can be monitored by Western Blot on SIGLEC3 immune-precipitation or whole cell lysate prepared from MDSCs. MDSC survival can be monitored to examine if apoptosis or G1 arrest is increased after treatment with ICA/ICT. Increased PP2A activity could be one of the mechanisms that contribute to the reduced MDSC accumulation induced by ICA/ICT.

PP2A's phosphatase activity can also be verified by using an *in vitro* phosphatase assay kit. In all assays involved in testing PP2A activity Forskolin, an activator of PP2A, can be included as a positive control and okadaic acid, an inhibitor, can be used as a negative control. S100A9/SIGLEC3 signaling can lead to PDE5 activation and PP2A inhibition, but upon treatment with ICA/ICT, or Forskolin, PP2A activity can increase. Particularly the phosphorylation on Ser92 of PDE5 can be decreased by PP2A, providing evidence to support the specific effect of ICA/ICT on MDSC. Alternatively, LV- or adenoviral vectors containing PP2A*ca* can be used to overexpress PP2A in MDSC. Over expression of PP2A*ca* in Namalwa cells, a lymphoma cell line, can activate PP2A signaling pathway. Therefore, overexpression of PP2A*ca* in MDSC can be used to validate the effect of ICA/ICT by examining phospho-PDE5, apoptosis, expression of S100A9/SLGLEC3, NO and inflammatory cytokines production, as mentioned above.

Since there may be variations due to the use of cells from healthy donors or patients with cancer, an alternative strategy can be to use SJCRH30 cells that express SIGLEC3. This strategy can be used to strengthen the approach by either overexpression of PDE5 or PP2A in these cells following treatment with ICA/ICT and their derivatives disclosed herein. These experiments of PDE5 overexpression can be used to demonstrate its capacity to overcome the ICA/ICT-induced PP2A activation. Conversely, by overexpressing PP2A the action of ICA/ICT can be enhanced through the down-regulation of PDE5 activation.

Each downstream pathway can be inhibited with specific inhibitors (JNK inhibitor SP600125, p38 inhibitor SB203580 or ERK1/ 2 inhibitor PD98059, or SHP1/2 inhibitor as well as chelating agents EDTA or EGTA and glycosylases to understand the role of Ca⁺⁺ and sugar moieties in S100A9 ligation) to assess if PDE5, PP2A, S100A9 and SIGLECs are related to other pathways crucial in inflammation, and to understand if there is cross-talk among different pathways or which pathways are more critical in the modulatory function of ICA and ICT on MDSC. The expression of both S100A9/SIGLEC3 and the activation of PDE5 and PP2A can be measured to assess which of these pathways links these two proteins together. The read-out from these experiments will be phospho-PDE5 and phospho-PP2A by Western Blot, SIGLEC 3 expression by flow cytometry and the production of downstream of suppressive cytokines and soluble mediators. The biological results can be further validated by pathway specific shRNA treatment, to confirm results and pinpoint the specifics of each component in ICA and ICT action. After silencing specific proteins and treating with ICA, ICT or their derivatives disclosed herein, the phosphorylation status of components of the NF-κB, Akt, p38 and STAT3 pathways can be measured by Western Blot or flow cytometry.

These results can be corroborated by modifying key amino acids important for PDE5 inhibition *in silico* to guide the selection of the best mutants of the PDE5 enzyme to study binding of ICT to the catalytic pocket as well as a model for understanding biochemical conformations that can change the affinity for PP2A in the phosphorylation site. Mutations can be made on each separate amino acid in the binding pocket: H613, D764, F820, and S663 of PDE5 based on their biochemical properties and the best change can be selected based on the *in silico* model that could prevent binding with little or no modification to the overall structure of PDE5. This can help select a mutant that will be tested *in vitro* using the Quick Change site-directed mutagenesis assay kit. These clones can be introduced using Lipofectamine LTX Plus into either FACS sorted MDSCs or IMC. The differences can be assessed as compared to those that have wild type over expression of PDE5. These cells can be treated with different doses of compound for different lengths of time after which point cytotoxicity/proliferation can be measured by flow cytometry, co-immune-precipitation of PP2A and PDE5 (wild type and mutants) can be measured to examine if PDE5-mutuants will affect PP2A binding to PDE5,iNOS expression and NO production can be measured, the phosphorylation of the key pathway components Iκb, p38, AKT and STAT3 can be measured by Western Blot, and the release of the cytokines IFNγ, TNFα, IL-10 and TGFβ can be measured by ELISA and Q-PCR.

### Example 8: Counteracting SIGLEC3-ITIM signaling by activating adaptor protein, DAP12 to improve the tumor microenvironment through induction of MDSC maturation

Receptors that associate with DAP12, which harbors an ITAM motif, can function as activating receptors that overcome the signals emanating from ITIM-bearing molecules. They are able to do so by recruiting Syk/Zap70, which can lead to PI-3K and MAPK/ERK activation. The final balance between the ITAM and ITIM can be determined and orchestrated by the specific function that is associated with each receptor. Some CD33-related SIGLECs, such as SIGLEC-H or SIGLEC-14 lack ITIMs and are thus can directly interact with DAP12. The importance of DAP12 is that it can partner with several receptor complexes and play a role in myeloid development. DAP12 is especially implicated in the maturation of stem cells into monocytes and it can promote DC maturation and survival. A hurdle in cancer is that there is significant accumulation of immature myeloid cells with the MDSC phenotype. The accumulation MDSCs and the over-expression of SIGLEC3 in these cells can result in an imbalance of ITIM and ITAM-mediated signaling, in which the SIGLEC3-ITIM signaling dominates over activation signals coming from DAP12 (ITAM signal). Thus, immature myeloid cells can be prevented from undergoing further differentiation and maturation. Therefore, activating the DAP12 pathway in order to induce myeloid differentiation and maturation can be a potential strategy to decrease MDSCs in tumor microenvironment and improve tumor immunity.

To accomplish this, two constitutive active forms of DAP12 were created and inserted into an adenovirus vector. After multiple analyses (biological assay, biochemical assay, and immunochemical evaluation and signaling assay etc), it was confirmed that these constructs can signal downstream targets and induce the biological function. Using AD293 cells, these two active forms of DAP12 (P19 and P23) were able to bind Syk70/Zap70 leading to ERK/MAPK activation (**Fig. 13**). One of the advantages of using these constructs is that active DAP12 can signal on its own, bypassing the need for an activating receptor. As shown in **Fig. 14****,** data was generated showing the feasibility of these constructs to promote immature DC maturation as shown by increased expression of myeloid maturation markers like CD80, CD83, CCR7 after infection for three days. These results suggest that active DAP12 can have the potential to drive maturation of immature myeloid cells, leading to either reduced accumulation of MDSCs or down-regulation of their suppressive function. These findings corroborate the rationale for conducting the experiments proposed in this aim to target the signaling pathway mediated by DAP12.

Adenoviral vectors are not suitable for long-term assays as they are toxic to the cells. Therefore, lentivirus vectors will be used *in vivo* tumor bearing mice with the capability of transducing non-dividing cells. DAP12 and DAP12 mutants can be converted from adenovirus into LV vectors. The DAP12 constructs can include wild type (wt-DAP12), dominant-negative DAP12 (dnDAP12) and two active forms of DAP12 (P19 and P23). LV with EGFP alone can also be used as a vector control and a way to monitor infection.

Because there can be reduced DAP12 mRNA expression in MDSCs isolated from patients with MDS/AML, it is possible that there can be low level expression or signaling deficiency in MDSCs from cancer patients. Therefore, gene expression of DAP12 in MDSCs isolated by FACS sorting from cancer can be evaluated by quantitative real-time PCR (Q-PCR) for DAP12 expression. In this study, immature myeloid cells from healthy donors can be used as a control. The signaling event of DAP12 can be analyzed by using mAb to cross-link a DAP12 associated activating receptor, TREM (triggering receptor expressed on myeloid cells), followed by immunoprecipitation with anti-DAP12 and Western Blot with anti-tyrosine phosphorylation antibody. Cross-linking of TREM in control cells can cause increased phosphrylation on tyrosine site of ITAM on DAP12 and can lead to increased recruitment of Syk as well as activation of PI-3 kinase and ERK, which can be readily detected by Western Blot as described in **Fig. 13****.** Meanwhile, the signaling event in MDSCs isolated from cancer patients can be decreased or inhibited when compared with control. The correlation coefficient can be calculated to determine if the reduction in DAP12 mRNA is correlated with functional deficiency. Both of these assays are reliable in detecting expression and function of DAP12. These experiments can demonstrate whether DAP12 is critical for immature myeloid differentiation. Additionally, it can provide information of any DAP12 deficiency (or other molecules in this pathway) that may exist in cancer patients.

The expression of maturation surface markers in MDSC can be analyzed after introducing DAP12 constructs. MDSCs from cancer patient can be infected with LV containing different mutant of DAP12 constructs. Cells can be harvested on days: 0, 3, 5 and 7 and their phenotypes can be analyzed by antibodies either for MDSC or for maturation markers (including CD80, CD83, CCR7, CD14, CD15 and HLA-DR). Over expression of DAP12 in patient's cells can induce a decrease in MDSC population and increase the mature cell population with monocyte or granulocyte maturation markers (CD14 and CD15, respectively). This phenotypic change can occur in a time-dependent fashion following introduction of active DAP12.

The suppressive function of MDSCs after transfection with active DAP12 can be examined. This can include examining the production of suppressive cytokines (TGGβ, IL10, VEGF, etc.) and generation of suppressive soluble mediators (ROS, NO and arginase production, etc.). Introduction of active DAP12 in MDSC can reduce MDSC suppressive function by blocking MDSC associated cytokines and soluble factor production.

Recognized functional properties of MDSCs include suppression of antigen stimulated or CD3 stimulated T cell proliferation and interferon-gamma (IFN-y) production. Therefore, T cell proliferation in response to anti-CD3/CD28 stimulation and IFN-γ production can be monitored by culturing autologous T cells with LV-DAP12 constructs infected MDSCs for 5-7 days before examining their proliferation by 3H-Thymidine incorporation and IFN-γ production by ELISA or ELISPOT.

S100A8/A9 expression level can also be determined by Western Blot analysis after over-expression of active DAP12, to assess any correlation between the S100A8/A9 signaling with DAP12 signaling pathways.

Over-expression of active DAP12 and its ability to inhibit SIGLEC3-ITIM mediated signaling following ligation with S100A9 or control ligand S100A7 can also be examined. To assess the ITIM signaling of SIGLEC3, phosphorylation of ITIM motifs or recruitment of cytoplasmic SHP1/2 can be measured by immunoprecipitation of SIGLEC3 receptor using anti- SIGLEC3 antibodies, followed by Western Blot analysis with anti-phosphotyrosine or anti-phosphatase antibodies, respectively. These experiments can be used to determine if the hyperactive CD33 signaling pathway in MDSC can be downregulated by over expression of active DAP12.

To further reduce expression of these inhibitory receptors, LV-shSIGLEC33 can be expressed in addition to the active DAP12. The combined role of these signaling molecules in modifying the tumor microenvironment in cancer patients can be examined. MDSCs isolated from cancer patients can be double infected with either LV-DAP12 or LV-shRNA to SIGLEC3 and these cells can be examined for their expression of maturation surface markers and their suppressive activities. This combined strategy can inhibit MDSC mediated activities and improve the tumor microenvironment.

Several CD33-r SIGLECs expression constructs, including SIGLEC 5 and SIGLEC 14 as well as their mutated forms, have been made that can be used to study both active and inhibit signals. Among them, SIGLEC 14 is an active SIGLEC by association with DAP12 to deliver the maturation signal. SIGLEC 14 and DAP12 have been co-transfected into AD293 cells before cross-linking with anti-SIGLEC 14 antibodies. As shown in **Fig. 15****,** cross-linking cells co-transfected with SIGLEC 14 and DAP12 suppressed the SIGLEC 3-ITIM pathway endogenous to these cells as indicated by reduced IL-10 production.

Little is known about DAP12 in MDSC maturation *in vivo,* especially whether they have a potential role in tumor development. Therefore, DAP12 knockout mice (DAP12^{-/-}) can be used to further validate the *in vitro* studies discussed above. Results indicate that DAP12-mediated signaling can be critical for NK cell activation and maturation and the lost NK function in these mice cannot be compensated by other ITAM-bearing adaptors. These data suggest that DAP12 can mediate a unique biological role when partnered with its specific activating receptors in these cells.

The percentages and expression of Gr-1⁺CD11b⁺ cells from the spleen and bone marrow of the DAP12 mice can be examined using flow cytometry based approaches. These experiments can determine whether DAP12 deficiency and the ensuing absence of DAP12-mediated activating signals can affect the maturation and/or accumulation of immature myeloid cells. Wild-type (wt), C57BL/6 mice, can be used as the control mice. Since inflammation and cancer is associated with the aging process, DAP12^{-/-} and wt-mice can be examined for Gr-1 and CD11b expression at different ages including 1, 3, 6, 9, 12, 15, 18, and 21 months. These experiments can address 1) whether MDSCs accumulate or expand in DAP12^{-/-} mice and 2) whether accumulation and expansion of MDSCs is accelerated by the aging process with DAP12 deficiency. Based on the *in vitro* data, there can be a blockage in immature myeloid cell differentiation and maturation in DAP12^{-/-} mice in contrast to wt-mice. Therefore, the immature myeloid cell population with Gr-1⁺CD11b⁺ surface marker expression can be accumulated and expanded in the bone marrow of DAP12^{-/-} mice. Further, increased MDSC numbers can be found in aged mice and this process can be accelerated in the absence of DAP12.

The following experiments can be used to evaluate if Gr-1⁺CD11b⁺ cells isolated from DAP12 mice are functioning as classical MDSCs that are found in malignancy and other pathological conditions.

The production of both suppressive cytokines and soluble suppressive mediators such as, TGF-β, IL-10, VEGF, arginase and NOS, can be monitored. Since active DAP12 expression was able to overcome the suppressive cytokine production mediated by SIGLEC3 or SIGLEC5 (ITIM bearing), the absence of DAP12 signaling can result in overproduction of suppressive mediators and cytokines due to an imbalance in inhibitory signaling emanating from SIGLEC3 or SIGLEC5.

The T cell response in DAP12^{-/-} mice can be examined. The lack of DAP12 signaling in the myeloid cells of the DAP12^{-/-} mice can prevent cells from differentiation/maturation, which can increase MDSC accumulation along with suppressive mediators and cytokine production, as well as display impaired T cell proliferation and reduced IFNγ production.

To further validate the specific role of DAP12 in MDSC development, an active form of DAP12 (the same active DAP12 constructs can be used in both human and mouse due to their high homology) can be introduced into MDSCs isolated from DAP12^{-/-} mice and can be used to assess whether elevated DAP12 expression or activated DAP12 is able to inhibit suppressive cytokine production (TGF-β, IL10 and VEGF), increase T cell response and/or drive MDSC differentiation/maturation by measure the maturation surface markers.

### Example 9: Regulatory mechanisms of ICA/ICT on the tumor microenvironment in tumor-bearing models

MDSC can be isolated from spleen or tumor tissue from the *in vivo* model discussed above. S100A9 immune-precipitation can be performed, followed by mass spectrometry to identify and confirm the murine SIGLECs involved. The binding receptors can be tested as described above for their human counterparts and co-transfected into a mouse NIH 3T3 (*in vitro* transfection model) with murine S100A9 and/or corresponding SIGLEC to corroborate them as ligands/receptor. The specific murine S100A9/SIGLEC binding and co-localization/internalization can be monitored by immunostaining. The specific signaling upon S100A9 and corresponding SIGLEC ligation can be biochemically assessed, including the examination of ITIM phosphorylation, SHP1/2 recruitment, PDE5 activation and its association with PP2A as well as their suppressive. These biochemical experiments can use MDSCs isolated from wild type (WT), S100A9 knockout (KO) and S100A9 transgenic mice (Tg).

Characterization of the tumor environment after treatment with ICA/ICT can be assessed in a mouse model, as previously described. Briefly, 6 to 8 week old female BALB/c mice can be inoculated subcutaneously (s.c.) in the flank with 5×10⁵ 4T1-Neu mammary carcinoma cells or murine Lewis lung carcinoma. Tumor growth can be monitored with bidirectional tumor measurements using calipers every 2-3 days and tumor volume calculated. Three doses of 12, 25 or 50 mg/kg ICA, ICT, or derivatives as disclosed herein can be given either orally or by intraperitoneal (i.p.) injection three times a week starting on day 7 after tumor inoculation until completion of the experiment. The control group can be given an equivalent amount of vehicle by either route. Tumor and spleen MDSCs can be collected.

The tumors can be either paraffin embedded or separated into immune cells and tumor cells for re-culture. From either source, isolated cells can be stained intracellularly with a prepared panel of antibodies to measure the phosphorylation of status of key pathways. A kinetic study with various doses of ICA/ICT or their derivatives disclosed herein and times can be used to monitor if PDE5 activity is suppressed by injection of ICA/ICT *in vivo.* PP2A activity can be monitored to confirm the role of ICA/ICT in the enhancement of their phosphatase activity *in vivo.* After treatment with ICA/ICT, the PDE5 activity can be inhibited and PP2A activity can be increased *in vivo* when compared with mice treated with vehicle. By tackling this bi-dentate signaling event, the MDSC activity can be suppressed and the tumor microenvironment can be improved. This can be validated by: measuring the percentage changes in MDSC in the spleens and local tumor to assess the shift of MDSC into normal mature myeloid cells after treatment with ICA/ICT; measuring if ICA/ICT can reduce the level of suppressive cytokines generated by MDSC; and testing if ICA/ICT can inhibit the production of soluble suppressive mediators from MDSC.

Inhibition of antigen (Ag) specific T cell responses can indicate MDSC activation. Therefore, Ag specific T cell responses can be tested using T cells isolated from the spleen of TRP-1 deficient mice. This model has been used to study suppression of immune responses in melanoma B16 cell tumors injected in C57BL/6 syngeneic mice. Vaccination of WT and TRP-1 deficient mice with TRP-1 antigen highlighted the ability of suppressive components to maintain tumor and therefore can provide a suitable model to study the modulation of MDSC suppression by ICA/ICT after challenging the mice with B16 melanoma. After injection, these mice can be treated with ICA/ICT and the specific T cell activation in response to specific TRP-1 antigen in either WT or TRP-1 deficient mice can be monitored by T cell proliferation and IFNγ production in CD8⁺ cells. The number of IFNy-producing cells in response to stimulation to the specific TRP-1-derived peptide and control peptides can be evaluated using ELISPOT. T cell proliferation can be evaluated by 3H-thymidine incorporation. CD11b⁺Gr1⁺ MDSCs can be isolated from either B16 injected WT or TRP-1 deficient mice and added at different ratios to T cells isolated from healthy mice in the presence of TRP-1-derived peptides or control peptides before evaluation of T cell proliferation or IFN-γ production. The Ag specific immune suppression by MDSC can be reduced in T cells co-cultured with MDSC that have been pretreated with ICA/ICT or their derivatives disclosed herein.

S100A9Tg mice have enhanced MDSC accumulation in tumor tissues, while S100A9KO mice have reduced levels of MDSC even in the tumor setting, which allows for assessment of the direct effects of ICA/ICT or their derivatives disclosed herein on the tumor with reduced MDSC population (Cheng P et al. J Exp Med. 2008;205(10):2235-49). This set of experiments can allow the functional role of S100A9 and MDSC in the mechanism of ICA and ICT tumor suppression *in vivo* to be defined. There can be a correlation between the S100A9/SIGLEC pathway and the maintenance of suppression at the tumor site and, therefore, the role they play in the function of ICA and ICT is assessed. Mononuclear cells (MNC) can be isolated from removed tumors, spleen or from the femurs and tibias of mice and enriched using a commercial kit (Miltenyi Biotec). They can then be used for phenotypic analysis and the quantity of MDSC can be assessed for expression of Gr-1⁺CD11b⁺. These cells can accumulate in tumor, spleen and peripheral blood when compared with S100A9KO and WT mice. To determine how MDSC are modulated after ICA and ICT treatment in the tumor of S100A9Tg, MDSCs can be purified from both S100A9Tg and control mice treated with DMSO or ICA/ICT and used to: (a) assess the production of cytokines by ELISA or flow, (b) assess the production of MDSC suppressive soluble factors (ROS and NO) and changes of expression on their regulatory genes ARG2 and NOS2, (c) assay PDE5 activity, and (d) assay PDE5 activity or PP2A phosphatase activity. The experiments can be done in parallel with WT and S100A9KO mice as controls. The MDSC mediated activity in S100A9Tg mice can have a greater reduction in their suppressive function after treatment with ICA/ICT compared with DMSO-treated mice or S100A9KO mice.

An adoptive transfer strategy can be used to directly reveal the involvement of SIGLECs, S100A9 and MDSC. This can be done by adoptive transfer of MDSC isolated from treated S100A9Tg or KO mice into its untreated counterpart. The specificity of S100A9 and MDSCs in mediating suppression in WT mice can be studied and it can be determined if the delay in tumor growth is dependent only on MDSC. FACS purified MDSC from S100A9Tg treated with ICA or ICT at the concentrations described above, can be transferred to WT syngeneic recipients by tail vein injection. Adoptive transfer of Gr-1⁺CD11b⁺MDSC from S100A9Tg mice expressing the CD45.1 genetic marker can be adoptively transferred into CD45.2 recipients to individually monitor the populations during development. MDSC mediated functional and immune suppressive activity can be assessed by MDSC expression, and their accumulation within the spleen. The matured myeloid cells (Gr-1⁻CD11c⁺DEC205⁺F4/80⁺) from WT CD45.2 mice and the Gr-1⁺CD11b⁺ cells from S100A9KO mice can be adoptively transferred into recipient mice as a negative control for S100A9. After injection, these mice can be followed monthly (kinetic study) and tested for MDSC expression in the spleen, MDSC-associated cytokine production, and immune suppression. In addition to the kinetic study, a variable number of MDSC can be transferred into recipients and analyzed for any immune suppressive functions, such as T cell proliferation in response to anti-CD3/CD28. This strategy can be used to quantify the engagement of donor MDSC from S100A9Tg mice. A higher percent of MDSC accumulation and cytokine production can be observed in the spleen of the recipients of adoptive transfer of Gr-1⁺CD11b⁺MDSC from S100A9Tg mice treated with DMSO. These recipients can display significant immune suppression indicated by increased suppressive cytokine production and decreased T cell proliferation. In contrast, recipients that receive MDSC from mice that were treated with ICA/ICT can display normal myeloid cell maturation and reduced immune suppression. In this setting, the reduced accumulation and expansion of CD11b⁺Gr-1⁺ MDSCs after ICA/ICT treatment can be detected by flow cytometric analysis in adoptively transferred WT mice and S100A9KO, respectively, from different treatments. Their production of cytokines and soluble factors by purified treated MDSC can be decreased, confirming their functionality.

The human lung adenocarcinoma (such as A549) or liver hepatocellular carcinoma (such as HepG2) cell line, can be grown in NSG immunodeficient mice in order to study the effects of ICT/ICA on MDSC function and on human tumor growth. The NSG (NOD/Shiscid IL2r^{-/-}) mouse model has outstanding capacity for human tumor transplant and multi-lineage hematopoietic reconstitution.

A pilot study can be performed to determine the growth curve of tumor xenograft and to obtain basic information regarding the pharmacological effect of ICA/ICT and their derivatives disclosed herein. Mice can be randomized into vehicle control and experimental groups (n=12) 5 to 7 days post tumor inoculation at doses of 12, 25 and 50 mg/kg once daily for 2 to 3 weeks. Twenty five days post injection of tumor cells, half of the mice can be sacrificed and used for various experiments. They can be used to measure tumor volumes and weight. MDSCs can be purified from the tumor tissue and their phospho-PDE5 and PP2A activity can be monitored by Western Blot to examine if there is blockade effect of ICA/ICT on their activation. Phospho-MAPK, phospho-AKT, phospho-STAT3 and phospho-NF-kB antibodies can be used to check if the compounds have an *in vivo* inhibitory effect on the inflammatory reaction of the human xenografts.

Immune reconstitution experiments can be performed to investigate the specific effects of ICA/ICT and their derivatives disclosed herein on MDSC and restore or enhance immune response. Briefly, 25 days post injection of tumor cells, human T cells and CD33⁺ myeloid cells can be given to the mouse by tail vein injection. It can take about two months for the successful engraftment of these myeloid cells. The mice can be divided into control and experimental groups as described above and receive vehicle, ICA or ICT once a day for 2 weeks and then used for various experiment. Flow cytometry can be used to examine the number of infiltrating human MDSC using the human MDSC surface markers: CD33+HLA-DR⁻LIN⁻ from single cell suspension of the tumor tissue. Mice treated with ICA/ICT can have lower numbers of CD33⁺HLA-DR⁻LIN⁻ expressing MDSC due to the inhibitory effects of ICT/ICA on MDSC expansion, possibly by ICT/ICA induced PP2A activation, and may consequently cause MDSC to undergo apoptosis or by promoting MDSC differentiation and maturation. Blood samples can be collected and plasma can be prepared to examine human TGF-β, IL-10 and VEGF production using specific ELISA kits. Inhibiting S100, SIGLEC and TLR signaling using ICA/ICT can inactivate MDSC and decrease their ability to generate suppressive cytokines. One portion of tumor tissue can be paraffin embedded to study the local infiltration of CD4⁺ or CD8⁺ T cells by immunostaining. T cells of human origin can be isolated either from tumor tissue, spleen, or peripheral blood and the IFN-γ producing cells can be determined by ELISPOT. T cells isolated from ICA, ICT or vehicle treated mice can be stimulated with anti-CD3/CD28 and a cell proliferation assay can be performed by measuring 3H-incorporation. T cell proliferation and the number of IFNγ producing cells can be significantly higher in ICT/ICA treated mice when compared with DMSO control treated mice.

An alternative strategy can be performed by measuring NK-mediated antibody dependent cytotoxicity (ADCC) on SIGLEC3⁺ MDSC. ICA can increase NK cell activity and lymphokine-activated killer cell (LAK) activity (0.1 to 1.0 microgram/ml) in both tumor patients and healthy donors (He W et al. Arzneimittelforschung. 1995;45(8):910-3). Recently, it was found that ICA/ICT are able to increase the expression level of Fc receptor (CD16, FcyRIII) in CD3-CD56⁺ NK cells after 48 hours treatment with the compounds (Fig. 16). This is particularly of interest, because these Fc receptors can be critical for the development of ADCC, a cytotoxic function of NK cells. This function can involve the activation of NK cells through the ligation of CD 16 with an antibody already bound to a target cell which can trigger the target cell to undergo apoptosis through the release of cytotoxic granules containing perforin and granzymes. Humanized anti-SIGLEC3/CD33 antibodies can be used to apply a strategy to deplete MDSC by NK-mediated ADCC.

To investigate whether targeting MDSC will restore immune response and improve the tumor microenvironment, the following experiments can be performed to test both the *in vitro* and *in vivo* efficacy of ICA/ICT induced NK-mediated ADCC on SIGLEC3⁺ target cells.

To measure NK-mediated ADCC *in vitro,* NK cells from peripheral blood of healthy donors can be isolated and treated with ICA/ICT or DMSO for 48 hours. CRL-2597, an endothelial cell line that cannot be killed by NK cells, can be transfected with SIGLEC3, labeled and used as a target in a chromium-51 [Cr⁵¹] following incubation with either anti-SIGLEC3 antibody or isotype IgG for 30 min on ice (Chen X et al. Blood. 2008). After co-culture with NK cells for 4 hours, [Cr⁵¹] release can be measured. ICA/ICT-treated NK cells can have increased ADCC against SIGLEC3 transfected CRL-2597 when compared with DMSO treated NK cells. Also, target cells cultured with anti-SIGLEC3 antibody can have more [Cr⁵¹] release when compared with target cells pre-incubated with isotype IgG.

Distinguishing functional criteria of described the MDSCs include suppression of antigen-stimulated or CD3-stimulated T cell proliferation and interferon-gamma (IFN-y) production. Therefore, to examine T cell responses after depletion of MDSC by NK ADCC, MDSCs can be isolated from the peripheral blood of cancer patients. After incubation with anti-SIGLEC3 antibodies or isotype IgG, these MDSC can be re-cultured with autologous PBMC already treated with or without ICA/ICT. After re-stimulation of TCR with anti-CD3/anti-CD28 for 5-7 days T cell proliferation can be monitored by Brdu incorporation and analyzed by flow cytometry on CD3⁺ T cells. IFN-γ production from these T cells can also be monitored by intracellular staining. Depletion of SIGLEC3⁺ MDSCs can improve T cell responses as compared to the isotype IgG and DMSO treated group due to the depletion of MDSCs by NK cells present in the PBMC with increased ADCC after treatment with ICA/ICT or their derivatives disclosed herein.

Following a similar setting, the suppressive cytokines, NO and ROS can be examined by ELISA. the production of these suppressive factors can be greatly reduced due to MDSC-depletion by NK mediated ADCC.

An *in vivo* approach can be employed by treating NK cells with or without compounds prior to administration of both NK cells and anti-SIGLEC3 antibodies into the xenograft tumor model as described above. NK cells treated with isotype IgG and NK cells treated with DMSO can be used as controls. The biological function of MDSC can be monitored as detailed above. The MDSC accumulation and expansion can be monitored. Immune suppression and tumor growth can be tested. Depletion of MDSC *in vivo* by NK mediated ADCC can improve immunity and tumor microenvironment, which can benefit the tumor bearing host by reducing tumor growth.

### Example 10:

ICT can down-regulate the expression of CD33 on PBMC (**Fig. 17**). The relative expression of SIGLEC3 by Q-PCR is shown in **Fig. 17A****.** Healthy PBMC were cultured for 48 hours with 20 µM of the PDE5 inhibitor ICT and the expression of SIGLEC3 measured by the DDCt method. The experimental control is PBMC treated with DMSO and the internal control was GAPDH. Error bars indicate the SEM of three separate donors/experiments. Flow cytometric analysis on the expression of CD33 on PBMC treated with 20 µM ICT is shown in **Fig. 17B****.**

ICT can down-regulate the expression of suppressive factors in PBMC (Fig. 18). The relative expression of IL-10, TGFb, TNFa, ARG2 and NOS2 by Q-PCR are shown in **Fig. 18****.** Healthy PBMC were cultured for 48 hours with 20 µM the gene expression of suppressive factors measured by the DDCt method. The experimental control is PBMC treated with DMSO and the internal control was GAPDH. Error bars indicate the SEM of three separate donors/experiments.

ICT can also down-regulate the expression of the suppressive cytokine IL-10 and TGFb on LPS-treated PBMC (**Fig. 19**). The relative expression of IL-10 and TGFb by Q-PCR are shown in **Fig. 19A** and **B**, respectively. Healthy PBMC were cultured for 48 hours with 20 µM ICT and the gene expression measured by the DDCt method. The experimental control is PBMC treated with DMSO for 48 hours and the internal control was GAPDH. Error bars indicate the SEM of three separate donors/experiments.

ICT can down-regulate the expression of CD33 and iNOS on MDS-BM (**Fig. 20**). The flow cytometric analysis on the expression of CD33 on PBMC treated with 20 µM ICT is shown in **Fig. 20A****.** The relative expression of CD33 in MDS-BM by Q-PCR is shown in **Fig. 20B****.** Healthy PBMC were cultured for 48 hours with ICA, ICT or the PDE5 inhibitor Sildenafil and the expression of SIGLEC3 measured by the Delta Delta Ct method. The experimental control is PBMC treated with DMSO for 48 hours and the internal control was GAPDH. Error bars indicate the SEM of three separate donors/experiments. The relative expression of NOS2 in MDS-BM was measured as described for B and is shown in **Fig. 20C****.**

The IC₅₀ values for ICT in various cell lines are shown in Table 1. The apoptosis rates for different doses of ICT in various cell lines are shown in Table 2.

**Table 1. IC₅₀ values for ICT in various cell lines.**

| **Cell line** | **IC₅₀ (µM) of ICT** |
|---|---|
| **B16** | 25.8 |
| **4T1** | 6.7 |
| **K562** | 50.5 |
| **Raji** | 25.6 |
| **MCF-7** | 23.2 |
| **Bone marrow** | > 100 |
| **U937** | > 100 |
| **PBMC** | > 100 |

**Table 2. Apoptosis rates of ICT in various cell lines.**

| **Cell line** | **Apoptosis rates (%)** | | |
|---|---|---|---|
| | **ICT 5µM** | **ICT 10µM** | **ICT 20 µM** |
| **B16** | 2.5 | 3.8 | 3.5 |
| **4T1** | 4.0 | 6.3 | 7.1 |
| **K562** | 5.5 | 6.7 | 7.8 |
| **Raji** | 9.1 | 9.2 | 10.0 |
| **MCF-7** | 3.9 | 4.1 | 6.1 |
| **Bone marrow** | 2.7 | 4.3 | 4.8 |
| **U937** | 3.1 | 2.6 | 2.7 |
| **PBMC** | 4.6 | 5.5 | 6.9 |

ICA and ICT can increase the hematopoiesis of MDS BMNCs (**Fig. 21**). Bone marrow mononuclear cells (BMCs) from MDS patients were treated with 20uM ICT for 48 h, and then seeded into MethoCult H4434 complete medium, the mixture is placed in 35-mm culture dishes (1 × 10⁵ cells/each dish) and incubated at 37°C in 5% CO₂ for 14 days. After incubation, colonies of CFU-GM and BFU-E were identified and counted using an inverted light microscope, as shown in **Fig. 21A** and **B** respectively. Each point represents the mean results of three normal individuals, and each experimental point represents duplicate plates. Results are expressed as mean value ± SD.

The docking model of ICT to PDE5A1 *in silico* is shown in **Fig. 22****.** A working 3D model of ICT was developed and retro-fitted into an available crystal structure of PDE5 using default values and removing all waters (2H44.pdb). Receptor grids were generated using the co-crystallized ligand Icarisid II, a metabolite of our compounds. Using LigPrep, Icarisid II was removed from 2H44.pdb and a library prepared for docking simulation (**Fig. 22A**). GLIDE provided docking scores (Gscores), representing an approximate binding of free energy of the ligand to the protein, using the previously generated grid. The pose that resembles the crystal structure pose from 2H44.pdb the best, has a glide Gscore of -13.676 Kcal/mol. For SP simulation, selected pose superimposes the co-crystal position of Icarisid II with an RMS of 0.3471 and forms a similar hydrogen bond network which includes the side chain of residue D764 and the backbone atom of residue 1665 with hydrogen in Icarisid II (**Fig. 22B**). A GLIDE extra precision (XP) docking simulation was conducted with the best pose from the SP docking results as the input ligand and the lowest energy pose (Gscore -17.215 Kcal/mol) closely approximates the co-crystal structure pose with an RMS of 0.9558 (**Fig. 22C**). The H-bond interactions for this pose occur between S668 with Icarisid II, H613 with Icarisid II and 1665 with Icarisid II, all of which are also present in the crystallographic pose. Superimposed crystal structure of Icarisid II with the XP dock pose of Icarisid II generated in the model (**Fig. 22D**). These results provide confidence that the model is suitable when used with XP, and that binding poses and interactions predicted for molecules related to Icarisid II will be reliable. Therefore, this model is appropriate for ligand design and optimization studies. ICT was generated by modifying Icaritin and an SP docking simulation executed, using the ICT LigPrep results (one structure only) as the ligand library and the receptor grid previously generated for PDE5A1 in the Icarasid II docking . Schrodinger's Sitemap 2.4 (Schrodinger, L.L.C.) was used to search for potential binding pockets in PDE5A1. For ICT, the pose with the best interactions has a glide Gscore of -10.764 Kcal/mol; there are four H-bond interactions between ICT and PDE5A1 in this pose: ICT and D764, ICT and Q817; ICT and S663; and ICT and H613. Also, this pose fits the hydrophobic regions of the sitemap results quite well as the crystal structure pose does.

ICA and ICT can inhibit the enzymatic activity of PDE5 (**Fig. 23**). The phosphodiesterase inhibition by ICA and ICT was measured with Promega's PDE-Glo system using the GMP-specific recombinant bovine phosphodiesterase Type V and used sildenafil at the same concentration as the active control as indicated in the manufacturer's instructions.

A scheme of PDE5 signaling and its link to PP2A and S100A9 is shown in **Fig. 24****.** GTP is converted to cGMP through the action of guanylil cyclase. PDE5 is then involved in the production of GMP which increases the levels of NO and more GC. This stimulates PKC which phosphorylate many signaling molecules, including PDE5. All of those processes lead to lower calcium, SIGLEC 3 and SIGLEC5 and S100A9 expression. S100A9 is known to regulate calcium and increase its concentration inside the cell which restarts the cycle. Conversely, activation of PP2A leads to the dephosphorylation and subsequent downregulation of those pathways including PDE5.

Other advantages which are obvious and which are inherent to the invention will be evident to one skilled in the art. It will be understood that certain features and sub-combinations are of utility and may be employed without reference to other features and sub-combinations. This is contemplated by and is within the scope of the claims. Since many possible embodiments may be made of the invention without departing from the scope thereof, it is to be understood that all matter herein set forth or shown in the accompanying drawings is to be interpreted as illustrative and not in a limiting sense.

Unless defined otherwise, all technical and scientific terms used herein have the same meanings as commonly understood by one of skill in the art to which the disclosed invention belongs. Publications cited herein and the materials for which they are cited are specifically incorporated by reference.

Those skilled in the art will recognize, or be able to ascertain using no more than routine experimentation, many equivalents to the specific embodiments of the invention described herein. Such equivalents are intended to be encompassed by the following claims.

### Preferred Embodiments

Embodiment 1. A compound having Formula I: 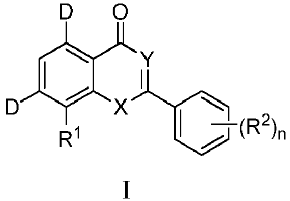 wherein
   Y is chosen from N, COH, COR, and CR¹;
   X is chosen from NH and O, with the proviso that when Y is N, X is NH and when Y is COH, COR, or CR¹, X is O;
   each D, independent of the other, is chosen from H, OH, OR, and halogen; R is alkyl or monoglucoside;
   R¹ is chosen from hydrogen, halogen, hydroxyl, amino, thiol, thioalkyl, alkyl, alkenyl, alkynyl, haloalkyl, cycloalkyl, heterocycloalkyl, alkylaryl, aryl, alkylheteroaryl, or heteroaryl, any of which is optionally substituted with acetyl, alkyl, amino, amido, alkoxyl, alkylhydroxy, cycloalkyl, heterocycloalkyl, aryl, heteroaryl, carbonyl, halogen, hydroxyl, thiol, cyano, or nitro;
   each R², independent of any other, is chosen from hydrogen, hydroxyl, alkoxyl, sulfonyl, amino, thiol, thioalkyl, alkyl, alkenyl, alkynyl, haloalkyl, cycloalkyl, heterocycloalkyl, alkylaryl, aryl, alkylheteroaryl, or heteroaryl, any of which is optionally substituted with acetyl, alkyl, amino, amido, alkoxy, alkylhydroxy, cycloalkyl, heterocycloalkyl, aryl, heteroaryl, carbonyl, halogen, hydroxyl, thiol, cyano, nitro, sulfonyl, or sulfonlylamino;
   n is 0, 1, 2, 3, 4 or 5;
   or a pharmaceutically acceptable salt or prodrug thereof,
   wherein X is not O, and Y is not COH or COR, and each D are not both OH or both OR, where R is a monosaccharide, and R¹ is not 3-methyl-2-butenyl, and R² is not n-methoxy.
Embodiment 2. The compound of embodiment 1, wherein each D is a hydroxyl group.
Embodiment 3. The compound of any of the embodiments 1-2, wherein Y is COH and X is O.
Embodiment 4. The compound of any of the embodiments 1-3, wherein n is 1.
Embodiment 5. The compound of embodiment 4, wherein R² is a methoxy group.
Embodiment 6. The compound of any of the embodiments 1-5 having Formula II: 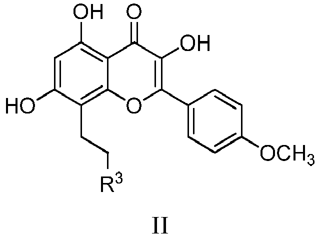
   wherein R³ is selected from hydrogen, halogen, hydroxyl, amino, alkyl, alkenyl, alkynyl, haloalkyl, cycloalkyl, heterocycloalkyl, alkylaryl, aryl, alkylheteroaryl, or heteroaryl, any of which is optionally substituted with carbonyl, alkyl, amino, amido, alkoxyl, alkylhydroxyl, cycloalkyl, heterocycloalkyl, aryl, heteroaryl, carbonyl, halogen, hydroxyl, thiol, cyano, or nitro;
   with the proviso that R³ is not chloroisopropyl, hydroxyisopropyl, isopropylacetamide, aminoisopropyl, methoxyisopropyl;
   or a pharmaceutically acceptable salt or prodrug thereof.
Embodiment 7. The compound of any of the embodiments 1-6, having Formula III: 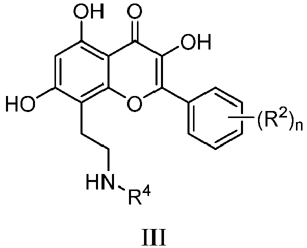
   wherein R⁴ is selected from alkyl, alkenyl, alkynyl, haloalkyl, cycloalkyl, heterocycloalkyl, alkylaryl, aryl, alkylheteroaryl, or heteroaryl, any of which is optionally substituted with carbonyl, alkyl, amino, amido, alkoxyl, alkylhydroxyl, cycloalkyl, heterocycloalkyl, aryl, heteroaryl, carbonyl, halogen, hydroxyl, thiol, cyano, or nitro;
   each R², independent of any other, is chosen from hydrogen, hydroxyl, alkoxyl, sulfonyl, amino, thiol, thioalkyl, alkyl, alkenyl, alkynyl, haloalkyl, cycloalkyl, heterocycloalkyl, alkylaryl, aryl, alkylheteroaryl, or heteroaryl, any of which is optionally substituted with acetyl, alkyl, amino, amido, alkoxy, alkylhydroxy, cycloalkyl, heterocycloalkyl, aryl, heteroaryl, carbonyl, halogen, hydroxyl, thiol, cyano, nitro, sulfonyl, or sulfonlylamino;
   n is 0, 1, 2, 3, 4 or 5;
   or a pharmaceutically acceptable salt or prodrug thereof.
Embodiment 8. The compound of any of the embodiments 1-7, having Formula IV: 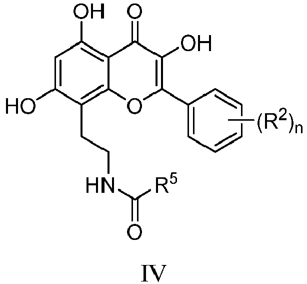
   wherein R⁵ is selected from alkyl, alkenyl, alkynyl, haloalkyl, cycloalkyl, heterocycloalkyl, alkylaryl, aryl, alkylheteroaryl, or heteroaryl, any of which is optionally substituted with carbonyl, alkyl, amino, amido, alkoxyl, alkylhydroxy, cycloalkyl, heterocycloalkyl, aryl, heteroaryl, carbonyl, halogen, hydroxyl, thiol, cyano, or nitro;
   each R², independent of any other, is chosen from hydrogen, hydroxyl, alkoxyl, sulfonyl, amino, thiol, thioalkyl, alkyl, alkenyl, alkynyl, haloalkyl, cycloalkyl, heterocycloalkyl, alkylaryl, aryl, alkylheteroaryl, or heteroaryl, any of which is optionally substituted with acetyl, alkyl, amino, amido, alkoxy, alkylhydroxy, cycloalkyl, heterocycloalkyl, aryl, heteroaryl, carbonyl, halogen, hydroxyl, thiol, cyano, nitro, sulfonyl, or sulfonlylamino;
   n is 0, 1, 2, 3, 4 or 5;
   or a pharmaceutically acceptable salt or prodrug thereof.
Embodiment 9. The compound of any of embodiments 1-5, having formula V: 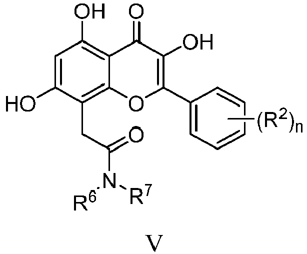
   wherein R⁶ and R⁷ are independently selected from alkyl, alkenyl, alkynyl, haloalkyl, cycloalkyl, heterocycloalkyl, alkylaryl, aryl, alkylheteroaryl, or heteroaryl, any of which is optionally substituted with carbonyl, alkyl, amino, amido, alkoxyl, alkylhydroxyl, cycloalkyl, heterocycloalkyl, aryl, heteroaryl, carbonyl, halogen, hydroxyl, thiol, cyano, or nitro;
   each R², independent of any other, is chosen from hydrogen, hydroxyl, alkoxyl, sulfonyl, amino, thiol, thioalkyl, alkyl, alkenyl, alkynyl, haloalkyl, cycloalkyl, heterocycloalkyl, alkylaryl, aryl, alkylheteroaryl, or heteroaryl, any of which is optionally substituted with acetyl, alkyl, amino, amido, alkoxy, alkylhydroxy, cycloalkyl, heterocycloalkyl, aryl, heteroaryl, carbonyl, halogen, hydroxyl, thiol, cyano, nitro, sulfonyl, or sulfonlylamino;
   n is 0, 1, 2, 3, 4 or 5;
   or a pharmaceutically acceptable salt or prodrug thereof.
Embodiment 10. The compound of any of embodiments 1-5, having Formula VI: 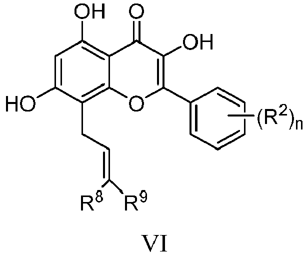
   wherein R⁸ and R⁹ are independently selected from alkyl, haloalkyl, cycloalkyl, heterocycloalkyl, alkylaryl, aryl, alkylheteroaryl, or heteroaryl, any of which is optionally substituted with carbonyl, alkyl, amino, amido, alkoxyl, alkylhydroxyl, cycloalkyl, heterocycloalkyl, aryl, heteroaryl, carbonyl, halogen, hydroxyl, thiol, cyano, or nitro;
   with the proviso that R⁸ and R⁹ cannot both be methyl;
   each R², independent of any other, is chosen from hydrogen, hydroxyl, alkoxyl, sulfonyl, amino, thiol, thioalkyl, alkyl, alkenyl, alkynyl, haloalkyl, cycloalkyl, heterocycloalkyl, alkylaryl, aryl, alkylheteroaryl, or heteroaryl, any of which is optionally substituted with acetyl, alkyl, amino, amido, alkoxy, alkylhydroxy, cycloalkyl, heterocycloalkyl, aryl, heteroaryl, carbonyl, halogen, hydroxyl, thiol, cyano, nitro, sulfonyl, or sulfonlylamino;
   n is 0, 1, 2, 3, 4 or 5;
   or a pharmaceutically acceptable salt or prodrug thereof.
Embodiment 11. The compound of embodiment 1, wherein Y is N and X is NH.
Embodiment 12. The compound of any of embodiments 1 or 11, wherein n is 2.
Embodiment 13. The compound of any of embodiments 1 or 11-12, having Formula VII: 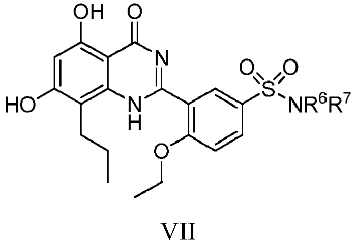 wherein R⁶ and R⁷ are independently selected from alkyl, alkenyl, alkynyl, haloalkyl, cycloalkyl, heterocycloalkyl, alkylaryl, aryl, alkylheteroaryl, or heteroaryl, any of which is optionally substituted with carbonyl, alkyl, amino, amido, alkoxyl, alkylhydroxyl, cycloalkyl, heterocycloalkyl, aryl, heteroaryl, carbonyl, halogen, hydroxyl, thiol, cyano, or nitro;
   or a pharmaceutically acceptable salt or prodrug thereof.
Embodiment 14. The compound of any of embodiments 1 or 11-12, having Formula VIII: 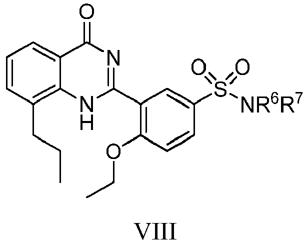 wherein R⁶ and R⁷ are independently selected from alkyl, alkenyl, alkynyl, haloalkyl, cycloalkyl, heterocycloalkyl, alkylaryl, aryl, alkylheteroaryl, or heteroaryl, any of which is optionally substituted with carbonyl, alkyl, amino, amido, alkoxyl, alkylhydroxyl, cycloalkyl, heterocycloalkyl, aryl, heteroaryl, carbonyl, halogen, hydroxyl, thiol, cyano, or nitro;
   or a pharmaceutically acceptable salt or prodrug thereof.
Embodiment 15. A pharmaceutical composition comprising a compound of any of the preceding embodiments.
Embodiment 16. A pharmaceutical composition comprising a therapeutically effective amount of a compound of Formula I: 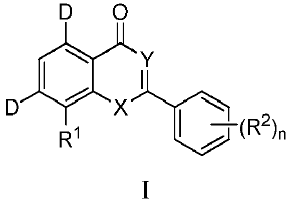 wherein
   Y is chosen from N, COH, COR, and CR¹;
   X is chosen from NH and O, with the proviso that when Y is N, X is NH and when Y is COH, COR, or CR¹, X is O;
   each D, independent of the other, is chosen from H, OH, OR, and halogen;
   R is alkyl or monoglucoside;
   R¹ is chosen from hydrogen, halogen, hydroxyl, amino, thiol, thioalkyl, alkyl, alkenyl, alkynyl, haloalkyl, cycloalkyl, heterocycloalkyl, alkylaryl, aryl, alkylheteroaryl, or heteroaryl, any of which is optionally substituted with acetyl, alkyl, amino, amido, alkoxyl, alkylhydroxy, cycloalkyl, heterocycloalkyl, aryl, heteroaryl, carbonyl, halogen, hydroxyl, thiol, cyano, or nitro;
   each R², independent of any other, is chosen from hydrogen, hydroxyl, alkoxyl, sulfonyl, amino, thiol, thioalkyl, alkyl, alkenyl, alkynyl, haloalkyl, cycloalkyl, heterocycloalkyl, alkylaryl, aryl, alkylheteroaryl, or heteroaryl, any of which is optionally substituted with acetyl, alkyl, amino, amido, alkoxy, alkylhydroxy, cycloalkyl, heterocycloalkyl, aryl, heteroaryl, carbonyl, halogen, hydroxyl, thiol, cyano, nitro, sulfonyl, or sulfonlylamino;
   n is 0, 1, 2, 3, 4 or 5;
   or a pharmaceutically acceptable salt or prodrug thereof,
   and a pharmaceutical carrier and optional anticancer or anti-inflammatory agent.
Embodiment 17. The pharmaceutical composition of embodiment 16, wherein the compound of Formula I is 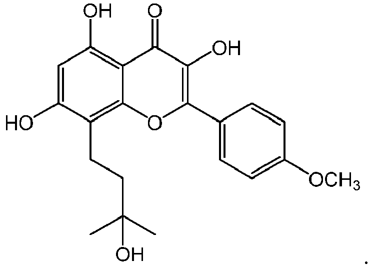
Embodiment 18. A method of treating myelodysplastic syndrome comprising:
   administering to the subject a therapeutically effective amount of a compound or composition of any one of the preceding embodiments.
Embodiment 19. A method of killing a tumor cell, comprising contacting a tumor cell with an effective amount of a compound or composition of any one of embodiments 1-17.

## Claims

1. A compound for use in treating myelodysplastic syndrome, wherein the compound is a compound of Formula II, III-A, V-A or VI-A: 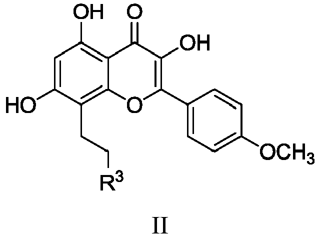 wherein R³ is an alkyl group optionally substituted with carbonyl, alkyl, amino, amido, alkoxyl, alkylhydroxy, cycloalkyl, heterocycloalkyl, aryl, heteroaryl, carbonyl, halogen, hydroxyl, thiol, cyano, or nitro; 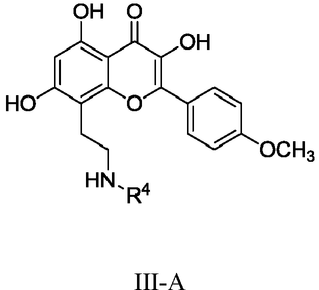 wherein R⁴ is selected from alkyl, alkenyl, alkynyl, haloalkyl, cycloalkyl, heterocycloalkyl, alkylaryl, aryl, alkylheteroaryl, or heteroaryl, any of which is optionally substituted with carbonyl, alkyl, amino, amido, alkoxyl, alkylhydroxy, cycloalkyl, heterocycloalkyl, aryl, heteroaryl, carbonyl, halogen, hydroxyl, thiol, cyano, or nitro; 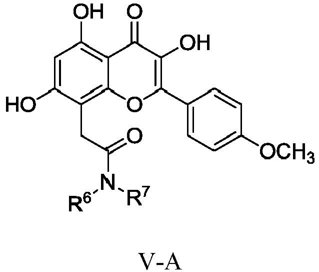 wherein R⁶ and R⁷ are independently selected from alkyl, alkenyl, alkynyl, haloalkyl, cycloalkyl, heterocycloalkyl, alkylaryl, aryl, alkylheteroaryl, or heteroaryl, any of which is optionally substituted with carbonyl, alkyl, amino, amido, alkoxyl, alkylhydroxy, cycloalkyl, heterocycloalkyl, aryl, heteroaryl, carbonyl, halogen, hydroxyl, thiol, cyano, or nitro; 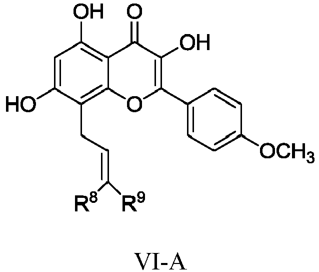
wherein R⁸ and R⁹ are independently selected from alkyl, haloalkyl, cycloalkyl, heterocycloalkyl, alkylaryl, aryl, alkylheteroaryl, or heteroaryl, any of which is optionally substituted with carbonyl, alkyl, amino, amido, alkoxyl, alkylhydroxy, cycloalkyl, heterocycloalkyl, aryl, heteroaryl, carbonyl, halogen, hydroxyl, thiol, cyano, or nitro;
with the proviso that R⁸ and R⁹ are not both be methyl.

2. A compound for use according to claim 1, wherein the compound is a compound of Formula II, wherein R³ is an alkyl group optionally substituted by carbonyl, alkoxyl, halogen, hydroxyl, or nitro.

3. A compound for use according to claim 2, wherein the compound is ICT

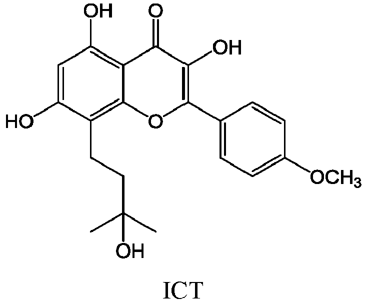

4. A compound for use according to claim 1, wherein the compound is a compound of Formula IV-A: 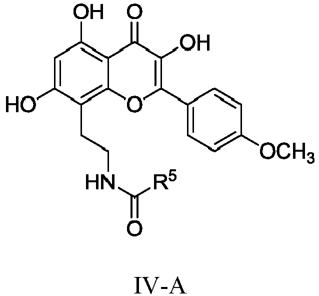 wherein R⁵ is selected from alkyl, alkenyl, alkynyl, haloalkyl, cycloalkyl, heterocycloalkyl, alkylaryl, aryl, alkylheteroaryl, or heteroaryl, any of which is optionally substituted with carbonyl, alkyl, amino, amido, alkoxyl, alkylhydroxy, cycloalkyl, heterocycloalkyl, aryl, heteroaryl, carbonyl, halogen, hydroxyl, thiol, cyano, or nitro.

5. A compound for use according to claim 3, wherein the compound decreases the accumulation and activation of myeloid derived suppressor cells.
